(19) Europäisches Patentamt
European Patent Office
Office européen des brevets

(11) EP 2 951 188 B1

(12) # EUROPÄISCHE PATENTSCHRIFT

(45) Veröffentlichungstag und Bekanntmachung des Hinweises auf die Patenterteilung:
**10.05.2017 Patentblatt 2017/19**

(21) Anmeldenummer: **14701708.1**

(22) Anmeldetag: **22.01.2014**

(51) Int Cl.:
***C07F 9/572*** (2006.01) ***C07F 9/6506*** (2006.01)
***C07B 43/04*** (2006.01) ***C07C 209/78*** (2006.01)
***C07C 211/08*** (2006.01) ***C07C 211/27*** (2006.01)
***C07C 211/35*** (2006.01) ***C07C 211/48*** (2006.01)
***C07C 213/00*** (2006.01) ***C07C 221/00*** (2006.01)
***C07C 225/16*** (2006.01) ***C07D 207/04*** (2006.01)
***C07D 209/08*** (2006.01) ***C07D 295/023*** (2006.01)
***C07D 295/03*** (2006.01) ***C07C 209/26*** (2006.01)
***C07D 295/033*** (2006.01) ***C07C 213/02*** (2006.01)
***C07F 9/145*** (2006.01)

(86) Internationale Anmeldenummer:
**PCT/EP2014/051178**

(87) Internationale Veröffentlichungsnummer:
**WO 2014/118038 (07.08.2014 Gazette 2014/32)**

(54) **PHOSPHOR-LIGANDEN UND VERFAHREN ZUR SELEKTIVEN RUTHENIUM-KATALYSIERTEN HYDROAMINOMETHYLIERUNG VON OLEFINEN**

PHOSPHORUS LIGANDS AND METHOD FOR THE SELECTIVE RUTHENIUM-CATALYZED HYDROAMINOMETHYLATION OF OLEFINS

LIGAND PHOSPHORÉ ET PROCÉDÉ D’HYDROAMINOMÉTHYLATION SÉLECTIVE D’OLÉFINES, CATALYSÉE PAR RUTHÉNIUM

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **01.02.2013 DE 102013201665**

(43) Veröffentlichungstag der Anmeldung:
**09.12.2015 Patentblatt 2015/50**

(73) Patentinhaber: **Evonik Degussa GmbH
45128 Essen (DE)**

(72) Erfinder:
- **FRANKE, Robert
45772 Marl (DE)**
- **DYBALLA, Katrin Marie
45657 Recklinghausen (DE)**
- **HESS, Dieter
45770 Marl (DE)**
- **FRIDAG, Dirk
45721 Haltern am See (DE)**
- **HAMERS, Bart
NL-5961 VG Horst (NL)**
- **BELLER, Matthias
18211 Nienhagen (DE)**
- **JACKSTELL, Ralf
27478 Cuxhaven Altenwalde (DE)**
- **FLEISCHER, Ivana
93057 Regensburg (DE)**
- **WU, Lipeng
City Xingtai
Hebei 055150 (CN)**

(56) Entgegenhaltungen:
**WO-A2-2004/101581 WO-A2-2007/078859
DE-A1- 10 321 421**

- **EDMOND LAM ET AL: "Reactions of 2-indolylphosphines with Ru3(CO)12: cluster capping with &#xFFFD;3,?2-indolylphosphine as an anionic six-electron P,N-donor ligand", DALTON TRANSACTIONS, Nr. 20, 1. Januar 2004 (2004-01-01), Seite 3383, XP055103804, ISSN: 1477-9226, DOI: 10.1039/b409930c**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- GUSTAVO ESPINO ET AL: "Synthesis and Structure of New (Polyphosphane)ruthenium Complexes with the Hemilabile Ligand 2-(Diphenylphosphanyl)-1-methyl-1H-imidazole- An Unexpected Rearrangement of [RuCl2(PN)(PPh3)2]", EUROPEAN JOURNAL OF INORGANIC CHEMISTRY, Bd. 2004, Nr. 12, 1. Juni 2004 (2004-06-01), Seiten 2542-2552, XP055103805, ISSN: 1434-1948, DOI: 10.1002/ejic.200300892
- CORINNA WETZEL ET AL: "Gold(I) Catalysts with Bifunctional P, N Ligands", INORGANIC CHEMISTRY, Bd. 50, Nr. 16, 15. August 2011 (2011-08-15), Seiten 7863-7870, XP055103806, ISSN: 0020-1669, DOI: 10.1021/ic2011259
- LIANG-HUA ZOU ET AL: "Metal-Free Phosphorylations of 1,3,4-Oxadiazoles and Related Heterocycles", SYNLETT, Bd. 23, Nr. 11, 13. Juni 2012 (2012-06-13) , Seiten 1613-1616, XP055103807, ISSN: 0936-5214, DOI: 10.1055/s-0031-1291150
- DENNIS PINGEN ET AL: "Direct Amination of Secondary Alcohols Using Ammonia", ANGEWANDTE CHEMIE INTERNATIONAL EDITION, Bd. 49, Nr. 44, 25. Oktober 2010 (2010-10-25), Seiten 8130-8133, XP055103812, ISSN: 1433-7851, DOI: 10.1002/anie.201002583
- STEPHEN S. MOORE ET AL: "Synthesis and coordinating properties of heterocyclic-substituted tertiary phosphines", THE JOURNAL OF ORGANIC CHEMISTRY, Bd. 47, Nr. 8, 1. April 1982 (1982-04-01), Seiten 1489-1493, XP055103801, ISSN: 0022-3263, DOI: 10.1021/jo00347a023
- JEROEN WASSENAAR ET AL: "INDOLPhos: novel hybrid phosphine-phosphoramidite ligands for asymmetric hydrogenation and hydroformylation", DALTON TRANSACTIONS, Nr. 34, 1. Januar 2007 (2007-01-01), Seite 3750, XP055103810, ISSN: 1477-9226, DOI: 10.1039/b709956h
- THOMAS SCHULZ ET AL: "Practical Imidazole-Based Phosphine Ligands for Selective Palladium-Catalyzed Hydroxylation of Aryl Halides", ANGEWANDTE CHEMIE INTERNATIONAL EDITION, Bd. 48, Nr. 5, 19. Januar 2009 (2009-01-19), Seiten 918-921, XP055103811, ISSN: 1433-7851, DOI: 10.1002/anie.200804898
- DAN-FU HU ET AL: "Preparation of New Buchwald-Type Secondary Phosphine Oxide Ligands and Applications in Suzuki-Miyaura Reactions", ORGANOMETALLICS, Bd. 30, Nr. 5, 14. März 2011 (2011-03-14), Seiten 1139-1147, XP055103844, ISSN: 0276-7333, DOI: 10.1021/om101132t
- CARINE MAALIKI ET AL: "On the P-Coordinating Limit of NHC-Phosphenium Cations toward Rh I Centers", CHEMISTRY - A EUROPEAN JOURNAL, Bd. 18, Nr. 25, 18. Juni 2012 (2012-06-18) , Seiten 7705-7714, XP055103846, ISSN: 0947-6539, DOI: 10.1002/chem.201104046
- DATABASE CA [Online] CHEMICAL ABSTRACTS SERVICE, COLUMBUS, OHIO, US; BAUMANN, THOMAS ET AL: "Copper(I) complexes, in particular for optoelectronic components", XP002720900, gefunden im STN Database accession no. 2012:1702168 & WO 2012/156378 A1 (CYNORA GMBH, GERMANY) 22. November 2012 (2012-11-22)
- IVANA FLEISCHER ET AL: "From Olefins to Alcohols: Efficient and Regioselective Ruthenium-Catalyzed Domino Hydroformylation/Reduction Sequence", ANGEWANDTE CHEMIE INTERNATIONAL EDITION, Bd. 52, Nr. 10, 12. Februar 2013 (2013-02-12), Seiten 2949-2953, XP055103803, ISSN: 1433-7851, DOI: 10.1002/anie.201207133
- LIPENG WU ET AL: "Efficient and Regioselective Ruthenium-catalyzed Hydro-aminomethylation of Olefins", JOURNAL OF THE AMERICAN CHEMICAL SOCIETY, Bd. 135, Nr. 10, 18. Februar 2013 (2013-02-18), Seiten 3989-3996, XP055103813, ISSN: 0002-7863, DOI: 10.1021/ja312271c

## Beschreibung

Die vorliegende Erfindung betrifft neue Phosphor-Liganden und ein chemo- und regioselektives Verfahren zur Hydroaminomethylierung von Olefinen mit mindestens einem primären oder sekundären Amin unter Zuführung von $H_2$ und CO unter katalytischen Bedingungen in Gegenwart eines Ruthenium-Komplexes und eines organischen Phosphor-Liganden.

Amine werden in einigen Millionen Tonnen pro Jahr produziert und finden z.B. als pharmakologisch und biologisch aktive Substanzen, als Farbstoffe, Agrochemikalien oder als Feinchemikalien industrielle Verwendung. Die zur Verfügung stehenden Synthesemethoden sind jedoch oft mit erheblichen Nachteilen verbunden. So sind z.B. die Ausgangssubstanzen für die Aminsynthese sehr teuer oder bei der Reaktion fällt eine große Menge an Nebenprodukten an oder zur Durchführung der Reaktion müssen Schutzgruppen eingeführt und wieder entfernt werden. Aus ökonomischen wie auch aus ökologischen Gründen besteht daher ein dringendes Bedürfnis, einfach umzusetzende Verfahren zur selektiven Herstellung aliphatischer Amine ausgehend von kostengünstigen Ausgangsmaterialien bereitzustellen.

Insbesondere die Synthese von Aminen aus an sich gut verfügbaren Olefinen ist von besonderem Interesse. Hier stellt die so genannte Hydroaminomethylierung eine umweltfreundliche Herstellung von Aminen dar. Seit der Entdeckung dieser Reaktion durch W. Reppe (Reppe, W.; Vetter, H. Liebigs Ann. Chem. 1953, 582, 133-163) wurde die Hydroaminomethylierungsreaktion weiter erforscht. Eine von L. F. Tietze (Chem. Rev. 1996, 96, 115-136) und G. H. Posner (Chem. Rev. 1986, 86, 831-844) beschriebene "Domino-Reaktion" umfasst die Hydroformylierung des Olefins zum Aldehyd, danach Aminierung des Aldehyds zum Enamin oder Imin sowie die anschließende Hydrierung des Zwischenprodukts:

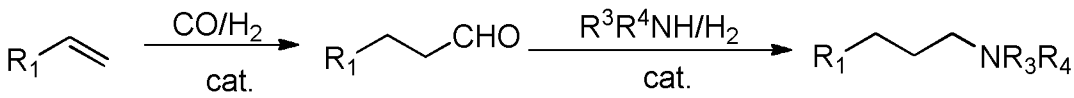


Trotz der Vorteile einer Hydroaminomethylierung, z.B. Verfügbarkeit der Edukte und Atomeffizienz, und trotz mancher Fortschritte in ihrer Anwendung wurden bislang vergleichsweise wenig synthetische Anwendungsmöglichkeiten für diese Reaktion bekannt.

In den letzten 15 Jahren wurden Hydroaminomethylierungsreaktionen intensiver im Bereich der organischen Chemie genutzt. Eilbracht et al. haben neue Varianten der Hydroaminomethylierungsreaktion entwickelt, mit denen zahlreiche pharmakologisch und synthetisch interessante Verbindungen synthetisiert werden konnten ((a) Eilbracht, P.; Kranemann, C. L.; Bärfacker, L. Eur. J. Org. Chem, 1999, 1907-1914. (b) Rische, T.; Bärfacker, L.; Eilbracht, P. Eur. J. Org. Chem, 1999, 653-660. (c) Koç, F.; Wyszogrodzka, M.; Eilbracht, P.; Haag, R. J. Org. Chem. 2005, 70, 2021-2025. (d) Beigi, M.; Ricken, S.; Mueller, K. S.; Koc, F.; Eilbracht, P. Eur. J. Org. Chem, 2011, 1482-1492. (e) Subhani, M. A.; Mueller, K.-S.; Eilbracht, P. Adv. Synth. Catal. 2009, 351, 2113-2123.)

Bislang wurden für diese Reaktionen im Allgemeinen einfache Rhodiumsalze oder eine Kombination von Rhodiumsalzen mit Triphenylphosphin als Katalysator eingesetzt. Bei Verwendung dieser Katalysatoren für die Hydroaminomethylierung von alpha-Olefinen besteht ein Hauptproblem in der nicht zufriedenstellenden Regioselektivität im ersten Reaktionsschritt. Ein weiterer Nachteil besteht in den geringen Katalysatoraktivitäten.

Die Gruppe um Beller entwickelte Rhodiumkatalysatoren, die modifizierte Naphos- and Xantphos-Liganden für die *n*-selektive Hydroaminomethylierung von Olefinen besitzen ((a) Seayad, A.; Ahmed, M.; Klein, H.; Jackstell, R.; Gross, T.; Beller, M. Science 2002, 297, 1676-1678. (b) Ahmed, M.; Seayad, A. M.; Jackstell, R.; Beller, M. J. Am. Chem. Soc. 2003, 125, 10311-10318. (c) Ahmed, M.; Bronger, R. P. J.; Jackstell, R.; Kamer, P. C. J.; van Leeuwen, P. W. N. M.; Beller, M. Chem. Eur. J. 2006, 12, 8979-88. (d) Ahmed, M.; Buch, C.; Routaboul, L.; Jackstell, R.; Klein, H.; Spannenberg, A.; Beller, M. Chem. Eur. J. 2007, 13, 1594-1601).

Zhang und seine Kollegen erreichten unter Verwendung von Tetrabi-typ Phosphorliganden in der Hydroaminomethylierungsreaktion gute Ergebnisse ((a) Liu, G.; Huang, K.; Cai, C.; Cao, B.; Chang, M.; Wu, W.; Zhang, X. Chem. Eur. J. 2011, 17, 14559-14563. (b) Liu, G.; Huang, K.; Cao, B.; Chang, M.; Li, S.; Yu, S.; Zhou, L.; Wu, W.; Zhang, X. Org. Lett. 2012, 14, 102-105). In diesen Arbeiten wurden Katalysatoren eingesetzt, die auf Rhodium und bidentaten P-Liganden mit großen Bisswinkeln (bite-angles) zur Kontrolle der Regioselektivität basieren. Die Menge des eingesetzten Liganden muss wesentlich erhöht im Vergleich zum Metall sein, d.h. ein hoher Ligandenüberschuss ist notwendig.

Über die Anwendung von billigeren Metallen wie Ruthenium wird kaum berichtet, da aus der Literatur bekannt ist, dass eine Ruthenium-katalysierte Hydroaminomethylierungsreaktion nicht besonders effizient ist, was durch die geringe Aktivität von Ruthenium im Hydroformylierungsschritt begründet ist ((a) Frediani, P.; Bianchi, M.; Salvini, A.; Carluccio, L. C.; Rosi, L. J. Organomet. Chem. 1997, 547, 35-40. (b) Hayashi, T.; Hui Gu, Z.; Sakakura, T.; Tanaka, M. J. Organomet. Chem. 1988, 352, 373-378. (c) Khan, M. M. T.; Halligudi, S. B.; Abdi, S. H. R. J. Mol. Catal. A: Chem. 1988, 48, 313-317. (d) Mitsudo, T.-a.; Suzuki, N.; Kondo, T.; Watanabe, Y. J. Mol. Catal. A: Chem. 1996, 109, 219-225).

In der DE 103 21 421 A1 wird ein chemo- und regioselektives Verfahren zur Hydroaminomethylierung von Alkenen beschreiben. Hierbei kommen bidentate Phosphor-Liganden zum Einsatz.

In der WO 2007/078859 A2 werden Liganden zur Hydroformylierung beschreiben. Bei den Liganden handelt es sich um Tetraphosphorliganden, welche in der Lage sind mehrfach an das Metallatom des Komplexes zu koordinieren.

Ein großes Problem der Hydroaminomethylierungsreaktionen stellt weiterhin auch eine langsame Hydrierung des intermediär entstehenden Enamins oder Imins dar. Wenn die Hydrierung nicht mit ausreichender Geschwindigkeit verläuft, können durch konkurrierende Aldolreaktionen unerwünschte Nebenprodukte entstehen. Für eine Hydroaminomethylierung muss das Metall, das als Katalysator genutzt wird, in 2 Schritten aktiv und selektiv sein, nämlich im Hydroformylierungs-und Hydrierungsschritt.

Aufgabe der vorliegenden Erfindung war es daher, ein Verfahren zur schnellen und hoch regioselektiven Hydroaminomethylierung von leicht zugänglichen Olefinen (Alkenen) zur Verfügung zu stellen, das insbesondere die oben genannten Nachteile vermeiden kann und kostengünstiger im Vergleich zum bisherigen Stand der Technik ist. Des Weiteren soll möglichst wenig *N*-Formylpiperidin im direkten Verfahrensprodukt vorliegen.

Die Aufgabe wird gelöst durch ein Verfahren gemäß Anspruch 1.

Verfahren zur Hydroaminomethylierung von Olefinen, dadurch gekennzeichnet, dass wenigstens ein Olefin mit mindestens einem primären oder sekundären Amin unter Zuführung von $H_2$ und CO in Gegenwart eines katalytischen Systems aus einem Ruthenium-Komplex und mindestens einem Liganden umgesetzt wird, wobei der Ligand einen organischen Phosphor-Liganden einer der allgemeinen Formeln (1) bis (8) darstellt:

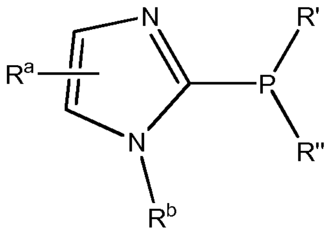
(1)

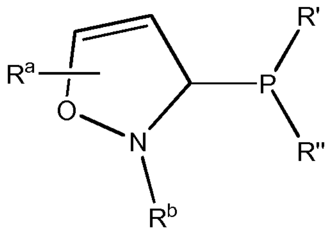
(5)

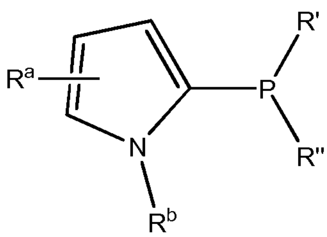
(2)

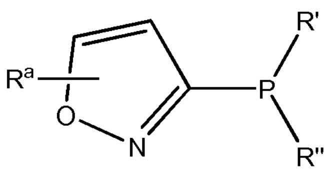
(6)

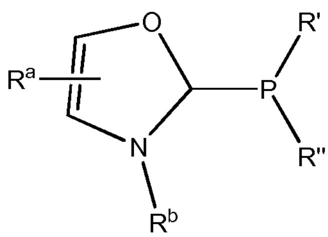
(3)

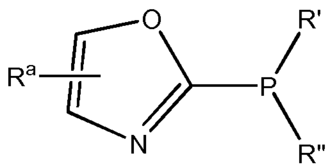
(7)

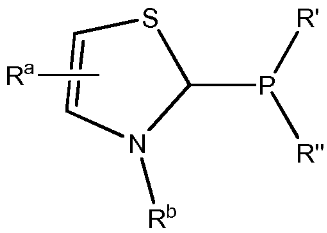
(4)

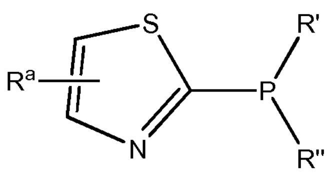
(8)

worin

R' und R" gleich oder verschieden sind und für einen Rest stehen, der ausgewählt ist aus der Gruppe bestehend aus:

($C_1$-$C_{12}$)-Alkyl, ($C_1$-$C_{12}$)-Heteroalkyl, ($C_4$-$C_{14}$)-Aryl, ($C_4$-$C_{14}$)-Aryl-($C_1$-$C_{12}$)-Alkyl, ($C_4$-$C_{14}$)-Aryl-O-($C_1$-$C_{12}$)-Alkyl, ($C_3$-$C_{14}$)-Heteroaryl, ($C_3$-$C_{14}$)-Heteroaryl-($C_1$-$C_{12}$)-Alkyl, ($C_3$-$C_{12}$)-Cycloalkyl, ($C_3$-$C_{12}$)-Cycloalkyl-($C_1$-$C_{12}$)-Alkyl, ($C_3$-$C_{12}$)-Heterocycloalkyl, ($C_3$-$C_{12}$)-Heterocycloalkyl-(C1-C12)-Alkyl, O-($C_1$-$C_{12}$)-Alkyl, O-($C_1$-$C_{12}$)-Heteroalkyl, O-($C_4$-$C_{14}$)-Aryl, O-($C_4$-$C_{14}$)-Aryl-($C_1$-$C_{14}$)-Alkyl, O-($C_3$-$C_{14}$)-Heteroaryl, O-($C_3$-$C_{14}$)-Heteroaryl-($C_1$-$C_{14}$)-Alkyl, O-($C_3$-$C_{12}$)-Cycloalkyl, O-($C_3$-$C_{12}$)-Cycloalkyl-($C_1$-$C_{12}$)-Alkyl, O-($C_3$-$C_{12}$)-Heterocycloalkyl, O-($C_3$-$C_{12}$)-Heterocycloalkyl-($C_1$-$C_{12}$)-Alkyl,

wobei die genannten Alkyl-, Heteroalkyl-, Cycloalkyl-, Heterocycloalkyl-, Aryl- und Heteroarylgruppen optional ein oder mehrfach substituiert sind und
$R^a$ und $R^b$ ausgewählt sind aus: Wasserstoff, Hydroxy, ($C_1$-$C_{12}$)-Alkyl, ($C_1$-$C_{12}$)-Heteroalkyl, ($C_4$-$C_{14}$)-Aryl, ($C_4$-$C_{14}$)-Aryl-($C_1$-$C_{12}$)-Alkyl, ($C_4$-$C_{14}$)-Aryl-O-($C_1$-$C_{12}$)-Alkyl, ($C_3$-$C_{14}$)-Heteroaryl, ($C_3$-$C_{14}$)-Heteroaryl-($C_1$-$C_{12}$)-Alkyl, ($C_3$-$C_{12}$)-Cycloalkyl, ($C_3$-$C_{12}$)-Cycloalkyl-($C_1$-$C_{12}$)-Alkyl, ($C_3$-$C_{12}$)-Heterocycloalkyl, ($C_3$-$C_{12}$)-Heterocycloalkyl-($C_1$-$C_{12}$)-Alkyl, O-($C_1$-$C_{12}$)-Alkyl, O-($C_1$-$C_{12}$)-Heteroalkyl, O-($C_4$-$C_{14}$)-Aryl, O-($C_4$-$C_{14}$)-Aryl-($C_1$-$C_{14}$)-Alkyl, O-($C_3$-$C_{14}$)-Heteroaryl, O-($C_3$-$C_{14}$)-Heteroaryl-($C_1$-$C_{14}$)-Alkyl, O-($C_3$-$C_{12}$)-Cycloalkyl, O-($C_3$-$C_{12}$)-Cycloalkyl-($C_1$-$C_{12}$)-Alkyl, O-($C_3$-$C_{12}$)-Heterocycloalkyl, O-($C_3$-$C_{12}$)-Heterocycloalkyl-($C_1$-$C_{12}$)-Alkyl,

wobei die genannten Alkyl-, Heteroalkyl-, Cycloalkyl-, Heterocycloalkyl-, Aryl- und Heteroarylgruppen optional ein oder mehrfach substituiert sind und
und wobei $R^a$ auch zur Bildung eines größeren kondensierten Ringes befähigt ist.

Alkyl steht für einen nicht verzweigten oder verzweigten aliphatischen Rest.
Aryl für aromatische (Kohlenwasserstoff-)Reste, vorzugsweise mit bis zu 14 C-Atomen, z. B. Phenyl- ($C_6H_5$-), Naphthyl- ($C_{10}H_7$-), Anthryl- ($C_{14}H_9$-), vorzugsweise Phenyl.
Cycloalkyl für gesättigte cyclische Kohlenwasserstoffe, die ausschließlich Kohlenstoff-Atome im Ring enthalten.
Heteroalkyl für einen nicht verzweigten oder verzweigten aliphatischen Rest, der ein bis vier, bevorzugt ein oder zwei, Heteroatome ausgewählt aus der Gruppe bestehend aus N, O, S und substituiertem N enthalten kann.

Heteroaryl für einen Arylrest, in dem ein bis vier, bevorzugt ein oder zwei, Kohlenstoffatome durch Heteroatome ausgewählt aus der Gruppe bestehend aus N, O, S und substituiertem N ersetzt sein können, wobei der Heteroarylrest auch Teil einer größeren kondensierten Ringstruktur sein kann.
Heterocycloalkyl für gesättigte cyclische Kohlenwasserstoffe, die ein bis vier, bevorzugt ein oder zwei, Heteroatome ausgewählt aus der Gruppe bestehend aus N, O, S und substituiertem N enthalten kann.
Unter Heteroarylrest, der Teil einer kondensierten Ringstruktur sein kann, werden bevorzugt Systeme verstanden, in denen kondensierte Fünf- oder Sechsringe gebildet werden, z.B. Benzofuran, Isobenzofuran, Indol, Isoindol, Benzothiophen, Benzo(c)thiophen, Benzimidazol, Purin, Indazol, Benzoxazol, Chinolin, Isochinolin, Chinoxalin, Chinazolin, Cinnolin, Acridin.

Die genannten substituierten N können einfach substituiert sein, die Alkyl-, Heteroalkyl-, Cycloalkyl-, Heterocycloalkyl-, Aryl- und Heteroarylgruppen können ein- oder mehrfach, besonders bevorzugt ein-, zwei- oder dreifach, substituiert sein durch Reste ausgewählt aus der Gruppe bestehend aus Wasserstoff, ($C_1$-$C_{14}$)-Alkyl, ($C_1$-$C_{14}$)-Heteroalkyl, ($C_4$-$C_{14}$)-Aryl, ($C_4$-$C_{14}$)-Aryl-($C_1$-$C_{14}$)-Alkyl, ($C_3$-$C_{14}$)-Heteroaryl, ($C_3$-$C_{14}$)-Heteroaryl-($C_1$-$C_{14}$)-Alkyl, ($C_3$-$C_{12}$)-Cycloalkyl, ($C_3$-$C_{12}$)-Cycloalkyl-($C_1$-$C_{14}$)-Alkyl, ($C_3$-$C_{12}$)-Heterocycloalkyl, ($C_3$-$C_{12}$)-Heterocycloalkyl-($C_1$-$C_{14}$)-Alkyl, $CF_3$, Halogen (Fluor, Chlor, Brom, Iod), ($C_1$-$C_{10}$)-Haloalkyl, Hydroxy, ($C_1$-$C_{14}$)-Alkoxy, ($C_4$-$C_{14}$)-Aryloxy, O-($C_1$-$C_{14}$)-Alkyl-($C_4$-$C_{14}$)-Aryl, ($C_3$-$C_{14}$)-Heteroaryloxy, N(($C_1$-$C_{14}$)-Alkyl)$_2$, N(($C_4$-$C_{14}$)-Aryl)$_2$, N(($C_1$-$C_{14}$)-Alkyl)(($C_4$-$C_{14}$)-Aryl), wobei Alkyl, Aryl, Cycloalkyl, Heteroalkyl, Heteroaryl und Heterocycloalkyl die vorgenannten Bedeutungen haben.

In einer Variante des Verfahrens sind R' und R" ausgewählt aus: ggf. substituierten ($C_4$-$C_{14}$)-ArylRest, einen ggf. substituierten unverzweigten oder verzweigten aliphatischen Kohlenwasserstoffrest oder einem ggf. substituierten Cycloalkylrest, und
$R^a$ und $R^b$ sind ausgewählt aus: Wasserstoff, ($C_1$-$C_{12}$)-Alkyl, ($C_1$-$C_{12}$)-Heteroalkyl, ($C_4$-$C_{14}$)-Aryl, ($C_4$-$C_{14}$)-Aryl-($C_1$-$C_{12}$)-Alkyl, ($C_4$-$C_{14}$)-Aryl-O-($C_1$-$C_{12}$)-Alkyl, ($C_3$-$C_{14}$)-Heteroaryl, ($C_3$-$C_{14}$)-Heteroaryl-($C_1$-$C_{12}$)-Alkyl, ($C_3$-$C_{12}$)-Cycloalkyl, ($C_3$-$C_{12}$)-Cycloalkyl-($C_1$-$C_{12}$)-Alkyl, ($C_3$-$C_{12}$)-Heterocycloalkyl, ($C_3$-$C_{12}$)-Heterocycloalkyl-($C_1$-$C_{12}$)-Alkyl, wobei die genannten Alkyl-, Heteroalkyl-, Cycloalkyl-, Heterocycloalkyl-, Aryl- und Heteroarylgruppen optional ein oder mehrfach substituiert sind
und wobei $R^a$ auch zur Bildung eines größeren kondensierten Ringes befähigt ist.

In einer anderen Variante des Verfahrens sind R' und R" ausgewählt aus: ($C_1$-$C_8$)-Alkyl, vzw. Methyl, Ethyl, Propyl, Isopropyl und tert. Butyl, O-($C_1$-$C_8$)-Alkyl- substituiertes-Phenyl, Aryl, vorzugsweise Phenyl, Cyclohexyl und
$R^a$ und $R^b$ sind ausgewählt aus: Wasserstoff, ($C_1$-$C_{12}$)-Alkyl, ($C_1$-$C_{12}$)-Heteroalkyl, ($C_4$-$C_{14}$)-Aryl, ($C_4$-$C_{14}$)-Aryl-($C_1$-$C_{12}$)-Alkyl, ($C_4$-$C_{14}$)-Aryl-O-($C_1$-$C_{12}$)-Alkyl, ($C_3$-$C_{14}$)-Heteroaryl, ($C_3$-$C_{14}$)-Heteroaryl-($C_1$-$C_{12}$)-Alkyl, (wobei die genannten Alkyl-, Heteroalkyl-, Cycloalkyl-, Heterocycloalkyl-, Aryl- und Heteroarylgruppen optional ein oder mehrfach substituiert sind

und wobei $R^a$ auch zur Bildung eines größeren kondensierten Ringes befähigt ist.

In einer anderen Variante des Verfahrens sind R' und R" ausgewählt aus: $C_1$ bis $C_8$-Alkyl (insbesondere Methyl, Ethyl, Propyl, Isopropyl und tert. Butyl), Phenyl, Cyclohexyl.

Eine Alkylgruppe hat bevorzugt 1-12, insbesondere 1 bis 6 Kohlenstoffatome. Alkylgruppen sind z.B. Methyl, Ethyl, Propyl, Isopropyl, 1-Butyl, tert. Butyl, 1-Pentyl, 1-Hexyl.

In einer Ausführungsform ist $R^b$ ausgewählt aus: Alkyl, Phenyl, ($C_4$-$C_{14}$)-Aryl-O-($C_1$-$C_{12}$)-Alkyl und Alkyl-substituiertes Phenyl.

In einer Ausführungsform ist $R^a$ gleich Wasserstoff.

In einer Variante des Verfahrens weist der Ligand die allgemeine Formel (1) oder (2) auf.

In einer Variante des Verfahrens weist der Ligand die allgemeine Formel (1) auf.

In einer Variante des Verfahrens weist der Ligand die allgemeine Formel (2) auf.

In einer Variante des Verfahrens ist R' ausgewählt aus: Cyclohexyl, iso-Propyl, Phenyl.

In einer Variante des Verfahrens ist R" ausgewählt aus: Cyclohexyl, iso-Propyl, Phenyl.

In einer Variante des Verfahrens sind R' und R" ausgewählt aus: Cyclohexyl, iso-Propyl, Phenyl.

In einer Variante des Verfahrens stehen R' und R" für den gleichen Rest.

In einer Variante des Verfahrens ist der Ligand ausgewählt aus der Gruppe bestehend aus:

2 -(Dicyclohexylphosphino)-1-(2-methoxyphenyl)-1H-imidazol (L1),
2 -(Dicyclohexylphosphino)-1-methyl-1H-imidazol (L2),
2-(Dicyclohexylphosphino)-1-phenyl-1H-imidazol (L3),
2-(Diisopropylphosphino)-1-(2-methoxyphenyl)-1H-imidazol (L4),
2-(Diphenylphosphino)-1-(2-methoxyphenyl)-1H-imidazol (L5),
2-(Dicyclohexylphosphino)-1-mesityl-1H-imidazol (L6),
2-(Dicyclohexylphosphino)-1-phenyl-1H-pyrrol (L7),
2-(Dicyclohexylphosphino)-1-(2-methoxyphenyl)-1H-pyrrol (L8),
2-(Dicyclohexylphosphino)-1-methyl-1H-benzo[d]imidazol (L9).

In einer Variante des Verfahrens wird das Verfahren als Eintopf-Verfahren durchgeführt.

In einer Variante des Verfahrens wird der Ruthenium-Katalysator in situ ausgehend von einem Vorkomplex gebildet, wobei als Rutheniumquelle Ruthenium-enthaltende Salze und Komplexe als Vorstufe verwendet werden, die Rutheniumcarbonylhydrid-Komplexe bilden, vorzugsweise Ru(0)-carbonylverbindungen, Ru(II)- und Ru(III)-halogenide.

In einer Variante des Verfahrens wird der Ruthenium-haltige Vorkomplex in Mengen von 0,001 bis 10 mol-% in Bezug auf die eingesetzte ethylenisch ungesättigte Verbindung eingesetzt.

In einer Variante des Verfahrens wird der Ligand equimolar oder im Überschuss zu Ruthenium eingesetzt, vorzugsweise in einem Verhältnis zwischen 1:1 bis 4:1.

In einer Variante des Verfahrens ist das Verhältnis zwischen gebildetem n- zu iso-Isomer im Amin-Endprodukt größer gleich 80:20, vorzugweise größer gleich 90:10.

In einer Variante des Verfahrens werden solche Alkene zur Umsetzung verwendet, die eine oder mehrere ethylenisch ungesättigte Doppelbindungen enthalten, wie $\alpha$-Alkene, interne lineare und interne verzweigte Alkene, Cycloalkene und Polyene.

In einer Variante des Verfahrens werden substituierte, olefinische Verbindungen zur Umsetzung verwendet, vorzugsweise handelt es sich hierbei um Carbonsäureester.

In dem Verfahren kann jede Art von ethylenisch ungesättigter Verbindung oder Mischungen davon mit jeder Art von primärem und/oder sekundärem Amin oder Mischungen davon umgesetzt werden.

Es war überraschend, dass bei Durchführung des Verfahrens sowohl mit einfachen terminalen Alkenen als auch mit internen linearen und verzweigten Alkenen sowie funktionalisierten Alkenen ein hohes regioselektives n/iso-Verhältnis erreicht werden kann, wobei der Ligand ein organischer Phosphin- oder Phosphitligand ist. Darüber hinaus konnten überraschenderweise die Alkene in dem Verfahren mit sehr hoher Effizienz und sehr hoher n-Selektivität einer Hydroaminomethylierung unterzogen werden.

Die phosphorhaltigen Liganden lassen sich gemäß dem Fachmann bekannten Syntheseverfahren, wie sie z. B. nach Methoden wie sie in Chemistry of Organophosphorous Compounds, Ed. F. R. Hartley, Serial Ed. S. Patai, Vol. 1, John Whiley, 1990 beschrieben sind, herstellen.

Die bevorzugt verwendeten 2-Phosphino-substituierten heterocyclischen Liganden können z.B. gemäß Zhang, H.; Cai, Q.; Ma, D. J. Org. Chem. 2005, 70 (13), 5164-5173 oder Schulz, T.; Torborg, C.; Schäffner, B.; Huang, J.; Zapf, A.; Kadyrov, R.; Börner, A.; Beller, M. Angew. Chem. Int. Ed. 2009, 48, 918-921 hergestellt werden.
So werden sie z.B. hergestellt, indem ein entsprechend substituierter Heterocyclus unter Rückfluss in THF gelöst wird. Unter Schutzgasatmosphäre und Kühlung (-30°C) wird n-Butyllithium (n-BuLi) in Hexan zugesetzt. Nach etwa 30 Minuten wird ein entsprechendes Dialkylchlorphosphin langsam zugegeben, auf Raumtemperatur erwärmt, weiter erwärmt auf

ca. 50°C und für ca. 1h gerührt. Nach Kühlung z.B. im Eisbad wird eine entgaste wässrige Ammoniumchloridlösung zugegeben, kurzzeitig gerührt und die organische Phase wird abgetrennt. Die wässrige Schicht wird mit Toluol extrahiert und die kombinierten organischen Schichten werden bei 45°C unter Vakuum eingeengt. Das Produkt wird aus Diethylether umkristallisiert und ergibt ein farbloses festes Produkt.

Die Liganden werden in der Regel als monodentate Phosphinliganden eingesetzt, die jedoch auch multidentat binden können (zum Beispiel über die Stickstoffatome oder andere Heteroatome).

Als Synthesegas im Prozess wird eine Mischung aus Kohlenmonoxid und Wasserstoff verwendet. Der Synthesegasgesamtdruck beträgt vorzugsweise 0,1 bis 10,0 MPa, insbesondere 0,5 bis 10,0 MPa und besonders bevorzugt 1,0 bis 8,0 MPa.

Die Zielreaktion läuft vorzugsweise bei Temperaturen von 60 bis 180 °C ab; besonders bevorzugt bei 100 bis 160 °C.

Erfindungsgemäß wird im vorliegenden Verfahren Ruthenium als Metallkatalysator eingesetzt. Der Ruthenium-Katalysator kann in situ ausgehend von einem Vorkomplex gebildet werden. Als Rutheniumquelle können alle Ruthenium-enthaltende Salze und Komplexe als Vorstufe verwendet werden, die unter den Reaktionsbedingungen Rutheniumcarbonylhydrid-Komplexe bilden. Beispielhaft genannt werden Ru(0)-carbonylverbindungen, Ru(II)- und Ru(III)-halogenide. Die Rutheniumverbindungen können in unterschiedlichen Oxidationsstufen vorliegen, die mit Synthesegas und P-enthaltenden Liganden zu entsprechenden aktiven Ruthenium-(hydrido)(carbonyl)-Komplexen reagieren.

Eine besonders bevorzugte Vorstufe zur Verwendung im erfindungsgemäßen Hydroaminomethylierungsverfahren ist Trirutheniumdodecacarbonyl $[Ru_3(CO)_{12}]$.

Um die gewünschten Katalysatorselektivitäten und Katalysatoraktivitäten zu erreichen, ist es notwendig die oben genannten Phosphor-enthaltenden Liganden zuzusetzen. Im vorliegenden Verfahren wird dieser Ligand equimolar oder im Überschuss zu Ruthenium eingesetzt. Das Verhältnis Ligand zu Ruthenium beträgt vorzugsweise zwischen 1:1 bis 50:1; in besonders bevorzugten Ausführungsformen zwischen 1:1 bis 4:1. Vorteilhafterweise wird der Ligand im leichten Überschuss zugegeben. Ganz besonders bevorzugt ist ein Verhältnis von Ligand:Ruthenium von 1,1:1.

Als Substrate für das erfindungsgemäße Verfahren können im Prinzip alle Verbindungen eingesetzt werden, die eine oder mehrere ethylenisch ungesättigte Doppelbindung enthalten, beispielsweise mehrfach ungesättigte Verbindungen und cyclische Alkene. Das sind z.B. Alkene wie $\alpha$-Alkene, interne lineare und interne verzweigte Alkene und schließt Cycloalkene und Polyene ein. Diese Alkene können mit jeder Art von primärem und/oder sekundärem Amin oder Gemischen zur Reaktion gebracht werden.

In einer bevorzugten Ausführungsform wird mindestens ein Alken ausgewählt aus Alken-Verbindungen der Formel (I)

$$H_2C=CR^1R^2 \qquad (I)$$

mit mindestens einem primären oder sekundären Amin ausgewählt aus Verbindungen der Formel (II)

$$R^3R^4NH \qquad (II)$$

zu mindestens einem Amin der Formel (III)

$$R^3R^4N\text{-}CH_2\text{-}CH_2\text{-}CR^1R^2 \qquad (III)$$

umgesetzt, wobei die Reste $R^1$, $R^2$, $R^3$ und $R^4$ unabhängig voneinander ausgewählt sind aus der Gruppe bestehend aus Wasserstoff, $(C_1\text{-}C_{14})$-Alkyl, $(C_1\text{-}C_{14})$-Heteroalkyl, $(C_4\text{-}C_{14})$-Aryl, $(C_4\text{-}C_{14})$-Aryl-$(C_1\text{-}C_{14})$-Alkyl, $(C_4\text{-}C_{14})$-Aryl-CO-$(C_4\text{-}C_{14})$-Aryl, $(C_3\text{-}C_{14})$-Heteroaryl, $(C_3\text{-}C_{14})$-Heteroaryl-$(C_1\text{-}C_{14})$-Alkyl, $(C_3\text{-}C_{12})$-Cycloalkyl, $(C_3\text{-}C_{12})$-Cycloalkyl-$(C_1\text{-}C_{14})$-Alkyl, $(C_3\text{-}C_{12})$-Heterocycloalkyl, $(C_3\text{-}C_{12})$-Heterocycloalkyl-$(C_1\text{-}C_{14})$-Alkyl, $CF_3$, $(C_4\text{-}C_{14})$-Alkyl-O-$(C_4\text{-}C_{14})$-Alkyl, $(C_4\text{-}C_{14})$-Alkyl-COO-$(C_4\text{-}C_{14})$-Alkyl, Alkenyl.

Alkyl, Cycloalkyl, Heteroalkyl, Aryl, Heteroaryl und Heterocycloalkyl haben die bereits oben genannten Bedeutungen.

Substituiertes N kann einfach substituiert sein, die genannten Alkyl-, Heteroalkyl-, Cycloalkyl-, Heterocycloalkyl-, Aryl- und Heteroarylgruppen können ein oder mehrfach, besonders bevorzugt ein-, zwei- oder dreifach, substituiert sein durch Reste ausgewählt aus der Gruppe bestehend aus Wasserstoff, $(C_1\text{-}C_{24})$-Alkyl, $(C_1\text{-}C_{24})$-Heteroalkyl, $(C_5\text{-}C_{14})$-Aryl, $(C_5\text{-}C_{14})$-Aryl-$(C_1\text{-}C_{24})$-Alkyl, $(C_5\text{-}C_{14})$-Heteroaryl, $(C_5\text{-}C_{14})$-Heteroaryl-$(C_1\text{-}C_{24})$-Alkyl, $(C_3\text{-}C_{12})$-Cycloalkyl, $(C_3\text{-}C_{12})$-Cycloalkyl-$(C_1\text{-}C_{24})$-Alkyl, $(C_3\text{-}C_{12})$-Heterocycloalkyl, $(C_3\text{-}C_{12})$-Heterocycloalkyl-$(C_1\text{-}C_{24})$-Alkyl, $CF_3$, Fluor, Chlor, Brom, Iod, $(C_1\text{-}C_{10})$-Haloalkyl, Hydroxy, $(C_1\text{-}C_{24})$-Alkoxy, $(C_5\text{-}C_{14})$-Aryloxy, $(C_5\text{-}C_{14})$-Heteroaryloxy, $N((C_1\text{-}C_{24})\text{-Alkyl})_2$, $N((C_5\text{-}C_{14})\text{-Aryl})_2$, $N((C_1\text{-}C_{24})\text{-Alkyl})(C_5\text{-}C_{14})$-Aryl und Nitro; $(C_1\text{-}C_{24})$-Alkyl-COO und $(C_5\text{-}C_{14})$-Aryl-COO. Alkyl, Cycloalkyl, Heteroalkyl, Aryl, Heteroaryl und Heterocycloalkyl haben die bereits genannten Bedeutungen.

Beispiele für erfindungsgemäß bevorzugte Alkylgruppen oder Heterocycloalkylgruppen sind Methyl-, Ethyl-, n-Propyl-, iso-Propyl-, n-Butyl-, iso-Butyl-, sec-Butyl-, t-Butyl-, Hexyl-, Cylopentyl-, Cyclohexyl-, Tetrahydrofuryl-, Tetrahydropyranyl-, Tetrahydrothiophenyl-, Piperidinyl- und Morpholinylreste.

Beispiele für erfindungsgemäß bevorzugte Arylgruppen oder Heteroarylgruppen sind Cyclopentadienyl-Anion-, Phenyl-, Naphthyl-, Pyrrolyl-, Imidazolyl-, Thiophenyl-, Pyridyl-, Pyrimidyl-, Indolyl-, Chinolinylreste, Carbazol, Piperidin, Morpholin, Piperazin, Thiomorpholin, Benzophenon, Cyclohexyl.

Bevorzugt handelt es sich bei $R^1$ und/oder $R^2$ um einen Rest ausgewählt aus der Gruppe bestehend aus: H, ($C_1$-$C_{10}$)-Alkyl, ($C_6$-$C_{10}$)-Aryl, ($C_4$-$C_{10}$)-Cycloalkyl, ($C_5$-$C_{10}$)-Heteroaryl, ($C_6$-$C_{10}$)-Aryl-($C_1$-$C_{10}$)-Alkyl, ($C_5$-$C_{10}$)-Heteroaryl-($C_1$-$C_{10}$)-Alkyl, ($C_6$-$C_{10}$)-Aryloxy, ($C_5$-$C_{10}$)-Heteroaryloxy, ($C_6$-$C_{10}$)-Aryloxy-($C_1$-$C_{10}$)-Alkyl, ($C_5$-$C_{10}$)-Heteroaryloxy-($C_1$-$C_{10}$)-Alkyl, ($C_1$-$C_{10}$)-Alkoxy-($C_1$-$C_{10}$)-Alkyl, Alkenyl.

Die Substituenten für $R^1$ sind vorzugsweise ausgewählt aus: H, Alkyl.

Die Substituenten für $R^2$ sind vorzugsweise ausgewählt aus: Hexyl, Propyl, Butyl, Octyl, Hexenyl, Butanoyl, Propanoyl, Phenyl, Benzyl.

Bei $R^3$ und/oder $R^4$ handelt es sich vorzugsweise um einen Rest ausgewählt aus der Gruppe bestehend aus: ($C_1$-$C_{10}$)-Alkyl, ($C_4$-$C_{14}$)-Aryl, ($C_3$-$C_{14}$)-Heteroaryl, Heteroalkyl, Cycloalkyl, ($C_4$-$C_{14}$)-Aryl-O-($C_4$-$C_{14}$)-Aryl. In einer bevorzugten Ausführungsform sind hierbei $R^3$ und $R^4$ kovalent miteinander verknüpft, so dass sich ein 5- bis 8-gliedriger, vorzugsweise 6-gliedriger Heterocyclus ausbildet, der neben dem sekundären N-Atom ein bis drei weitere Heteroatome, vorzugsweise ein weiteres Heteroatom, ausgewählt aus der Gruppe bestehend aus S, O und N enthalten kann, wobei falls ein weiteres N-Atom im Heterocyclus vorhanden ist, dieses vorzugsweise einen Substituenten ausgewählt aus der Gruppe bestehend aus ($C_1$-$C_{10}$)-Alkyl, ($C_5$-$C_{14}$)-Aryl und ($C_5$-$C_{14}$)-Aryl-($C_1$-$C_8$)-Alkyl, ($C_1$-$C_{18}$)-Alkyl-COO, -OH trägt. Auch kondensierte Ringe sind möglich.

Bei den erfindungsgemäß umgesetzten Aminen handelt es sich hierbei besonders bevorzugt um Alkyl-, Cycloalkyl-, Dialkyl-, Phenyl- oder Dibenzylamine, Piperidin, Morpholin, Thiomorpholin, Indolin oder N-substituiertes Piperazin, Pyrrolidin oder Aminoalkohole, Carboxylat.

Im erfindungsgemäßen Verfahren werden Lösungsmittel für den Katalysator eingesetzt. Als Lösungsmittel werden im Allgemeinen polare inerte organische Lösungsmittel oder/und Wasser verwendet. Beispielhaft genannt werden dipolar aprotische Lösungsmittel, aliphatische Ether, Amide, aromatische Verbindungen, Alkohole und Ester, Ether sowie deren Gemische. In bevorzugten Ausführungsformen handelt es sich bei dem Lösungsmittel um beispielsweise N-Methyl-2-pyrrolidon (NMP), Methanol, Ethanol, Toluol, Tetrahydrofuran (THF) und Propylencarbonat oder Gemische derselben, insbesondere um eine Mischung eines Alkohols mit einem Aromaten. Besonders bevorzugt ist eine Mischung aus Methanol und Toluol.

Der Katalysator kann auch in trägergebundener Form eingesetzt werden, wobei Alumosilicate, Siliciumdioxide wie Kieselgele oder Kieselgure, Aktivkohle, Aluminiumoxide, Titanoxide oder Zeolithe als Trägermaterialien zu nennen sind.

Eine Variante des erfindungsgemäßen Verfahrens zeichnet sich vorzugsweise dadurch aus, dass der Umsatz der Reaktion größer gleich 80 %, besonders bevorzugt größer gleich 90 % ist.

Eine Variante des erfindungsgemäßen Verfahrens zeichnet sich vorzugsweise ferner dadurch aus, dass die Selektivität bezüglich der Bildung des Amins ausgehend vom Alken größer gleich 60%, besonders bevorzugt größer gleich 90% beträgt.

Eine Variante des erfindungsgemäßen Verfahrens zeichnet sich vorzugsweise ferner dadurch aus, dass die Turnover-Frequenz (TOF) der Reaktion größer gleich 100, besonders bevorzugt größer gleich 200, vor allem größer gleich 250 beträgt.

Bei dem erfindungsgemäßen Verfahren können Turnover-Frequenzen [(TOF) = (mol Produkt / (mol katalytisch aktiver Komplex x Reaktionszeit)] der Katalysatoren in der Größenordnung von > 250 und mehr realisiert werden. Daher werden typischerweise zwischen 0,5 und 0,001 mol% Ruthenium - bezogen auf das Ausgangssubstrat - eingesetzt.

Aufgrund der signifikant verbesserten Katalysatoraktivitäten ist es bei dem erfindungsgemäßen Verfahren möglich, kleine Mengen an Katalysator zu verwenden, die den Prozess ökonomisch relevant machen.

Das erfindungsgemäße Verfahren ist insofern besonders überraschend und neu, da in der Vergangenheit keine hochselektiven Hydroaminomethylierungen von Olefinen mit Ruthenium-Komplexen mit ausreichender Aktivität beschrieben wurden. Das beschriebene Verfahren zeigt hier erstmals, dass unter den erfindungsgemäßen Bedingungen einfache und funktionalisierte Olefine mit unterschiedlichen Aminen erfolgreich umgesetzt werden können, wobei hohe Ausbeuten (bis zu 96%) und eine hervorragende Regioselektivität (n/iso bis 99:1) erreicht werden.

Dieses neue Verfahren zeichnet sich neben den bereits erwähnten Vorteilen ferner dadurch aus, dass Ruthenium als Katalysatormetall im Vergleich zum Rhodium derzeit deutlich kostengünstiger ist und somit eine wirtschaftlich interessante Alternative zur klassischen Rhodiumchemie darstellt.

<u>Ausführungsbeispiele</u>

<u>Allgemeine Verfahrensvorschrift für die Herstellung der Liganden $L_1$-$L_9$:</u>

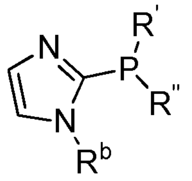

**$L_1$** $R^b$ = 2-OMe($C_6H_4$); R',R" = Cy
**$L_2$** $R^b$ = Me; R',R" = Cy
**$L_3$** $R^b$ = $C_6H_5$; R',R" = Cy
**$L_4$** $R^b$ = 2-OMe($C_6H_4$); R',R" ='Pr
**$L_5$** $R^b$ = 2-OMe($C_6H_4$); R',R" = Ph
**$L_6$** $R^b$ = 2,4,6-$Me_3$($C_6H_2$); R',R" = Cy

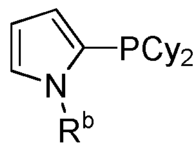

**$L_7$** $R^b$ = $C_6H_5$
**$L_8$** $R^b$ = 2-OMe($C_6H_4$)

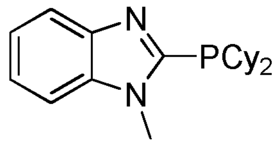

**$L_9$**

Ein entsprechend substituiertes -1*H*-Imidazol (13,5 mmol) wird unter Rückfluss in THF unter Argon gelöst und auf -30°C gekühlt. nBuLi (1,6 M in Hexan, 8,4 ml, 13 mmol) wird zugesetzt und das Reaktionsgemisch wird bei -30°C 30 Minuten lang gerührt. Danach wird eine Lösung des entsprechenden Dialkylchlorphosphin (14,8 mmol in 10 ml THF) langsam mittels Tropftrichter zugegeben (bei -30°C), auf Raumtemperatur erwärmt, weiter erwärmt auf ca. 50°C und für 60 Minuten gerührt. Nach Kühlung in einem Eisbad wird eine entgaste wässrige Ammoniumchloridlösung zugegeben, kurzzeitig gerührt und die organische Phase wird abgetrennt. Die wässrige Schicht wird mit Toluol (2 x 20 ml) extrahiert und die kombinierten organischen Schichten werden bei 45°C unter Vakuum eingeengt. Das Produkt wird aus Diethylether umkristallisiert und ergibt ein farbloses festes Produkt.

a) Herstellung von 2-(Dicyclohexylphosphino)-1-(2-methoxyphenyl)-*1H*-imidazol **[$L_1$]**

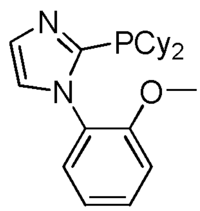

Entsprechend der obigen allgemeinen Vorschrift wurde **$L_1$** aus 1-(2-Methoxyphenyl)-*1H*-imidazol (2,35 g, 13,5 mmol) and Dicyclohexylchlorphosphin (3,43 g, 1,8 mmol) synthetisiert. Es wurde ein farbloser Feststoff erhalten (3,40 g, 68% Ausbeute).

b) Herstellung von 2-(Dicyclohexylphosphino)-1-methyl-1*H*-imidazol **[$L_2$]**

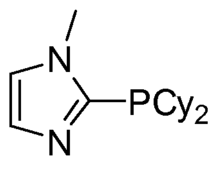

Entsprechend der obigen allgemeinen Vorschrift wurde **$L_2$** aus *N*-Methylimidazol (1,1 ml, 1,1 g, 14 mmol),

TMEDA (Tetramethylethylendiamin, 3,0 mL, 2,3 g, 20 mmol) und Chlordicyclohexylphosphin (3,0 ml, 3,2 g, 14 mmol) synthetisiert. Es wurde ein farbloses Öl erhalten, was nach Stehen erstarrte (2,14 g, 55% Ausbeute).
Siedepunkt 140 °C ($10^{-3}$ bar)

c) Herstellung von 2-(Dicyclohexylphosphino)-1-phenyl-*1H*-imidazol **[$L_3$]**

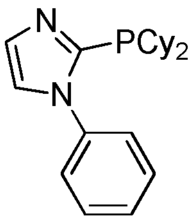

Entsprechend der obigen allgemeinen Vorschrift wurde **$L_3$** aus 1-Phenyl-*1H*-imidazol (1,95 g, 13,5 mmol) und Dicyclohexylchlorphosphin (3,43 g, 14,8 mmol) synthetisiert. Es wurde ein farbloser Feststoff erhalten (3,45 g, 75% Ausbeute).

d) 2-(Diisopropylphosphino)-1-(2-methoxyphenyl)-*1H*-imidazol [**$L_4$**]

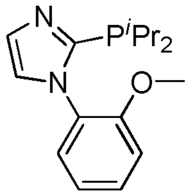

Entsprechend der obigen allgemeinen Vorschrift wurde **$L_4$** aus 1-(2-Methoxyphenyl)-*1H*-imidazol (2,35 g, 13,5 mmol) and Diisopropylchlorphosphin (2,25 g, 14,8 mmol) synthetisiert. Es wurde ein farbloser Feststoff erhalten (2,75 g, 70% Ausbeute).

e) Herstellung von 2-(Diphenylphosphino)-1-(2-methoxyphenyl)-*1H*-imidazol **[$L_5$]**

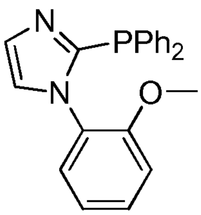

Entsprechend der obigen allgemeinen Vorschrift wurde **$L_5$** aus 1-(2-Methoxyphenyl)-*1H*-imidazol (2,35 g, 13,5 mmol) und Diphenylchlorphosphin (3,25 g, 14,8 mmol) synthetisiert. Es wurde ein farbloser Feststoff erhalten (3,82 g, 79% Ausbeute).

f) Herstellung von 2-(Dicyclohexylphosphino)-1-methyl-*1H*-benzo[d]imidazol **[$L_9$]**

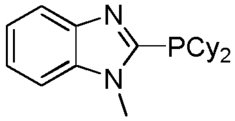

Entsprechend der obigen allgemeinen Vorschrift wurde $\mathbf{L_9}$ aus 1-Methyl-*1H*-benzo[d]imidazol (1,78 g, 13,5 mmol) und Dicyclohexylchlorphosphin (3,43 g, 14,8 mmol) synthetisiert. Es wurde ein farbloser Feststoff erhalten (3,10 g, 70% Ausbeute).

Die Herstellung der Liganden **[$\mathbf{L_6}$]** bis **[$\mathbf{L_8}$]** kann analog erfolgen.

**Allgemeine Verfahrensvorschrift für die Hydroaminomethylierung**

Es wird ein 100-ml Autoklav eingesetzt. Dieser wird mit [$Ru_3(CO)_{12}$](0,1 Mol%) und Ligand (0,33 Mol%), Olefin (20 mmol), Amin (24 mmol) sowie Lösungsmittel (20ml) unter Argonatmosphäre befüllt und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav auf Raumtemperatur abgekühlt und Gas abgelassen. Der Inhalt wird unter Argon überführt und nach der Zugabe des internen Standards (Isooktan) wird die Reaktionsmischung gaschromatographisch analysiert. Nach der Zugabe des internen Standards (Isooktan, 2,0 mL; 1,4 g; 12 mmol) wird die Reaktionsmischung nach Verdünnung mit Aceton mittels eines HP 6890 Gaschromatographs auf einer 30 m HP5 Säule analysiert. Hierfür wird folgende Methode verwendet: 10 Minuten bei 35 °C, dann heizen bis 280 °C mit 8 °C/min Aufheizrate, 6 Minuten bei 280 °C. Die Retentionszeiten einzelner Reaktanten und Produkten sind: 1-Okten (6,2 min), cis/trans 2-Oktene (6,9 min und 7,4 min), cis/trans 3- und 4-Oktene (6,0 min; 6,3 min und 6,5 min), Oktan (6,5 min), 1-Nonanal (19,5 min), isomere C9-Aldehyde (18,3 min; 18,5 min und 19,1 min), 1-Nonanol (21,1 min), isomere C9-Alkohole (19,8 min; 20,1 min und 20,2 min). Die Ausbeuten einzelner Produkte werden mit Hilfe der "Multiple Point Internal Standard GC Quantitation Method" bestimmt. Die Masse des Analyts wird mittels Gleichung (i) berechnet:

$$\text{Masse (Analyt)} = [\text{Masse (int. Standard)} \times \text{Signalfläche (int. Standard)} \times \text{Response-faktor}] / \text{Signalfläche (Analyt)} \qquad \text{(i)}$$

Der Responsefaktor wird über eine Kalibrierreihe mit 4 Analyt/Standard Lösungen mit verschiedenen bekannten Analyt- und Standardverhältnissen ermittelt. Für jede der Lösungen wird Responsefaktor als Verhältnis des Signals von Analyt (A) und Internem Standard (IS) auf bestimmte Konzentration normiert. Aus diesen Einzelwerten geht der Responsefaktor als deren Mittelwert hervor.

Die TON-(turnover number) und TOF-Werte (turnover frequency) werden mit den berechneten Ausbeuten mittels Gleichungen (ii) und (iii) bestimmt.

$$\text{TON} = \text{(Stoffmenge Produkt)/(Stoffmenge Katalysator)} = \text{Ausbeute*(Stoffmenge Substrat)/(Stoffmenge Katalysator)} \qquad \text{(ii)}$$

$$\text{TOF} = \text{TON/Reaktionszeit} \qquad \text{(iii)}$$

wobei man unter Reaktionszeit die Zeit versteht, die zwischen dem Anfang des Gasverbrauchs und dem Anfang des Abkühlens liegt.

**Erfindungsgemäße Beispiele 1 bis 7**

Ruthenium katalysierte Hydroaminomethylierung von 1-Octen mit Piperidin nach folgendem Reaktionsschema:

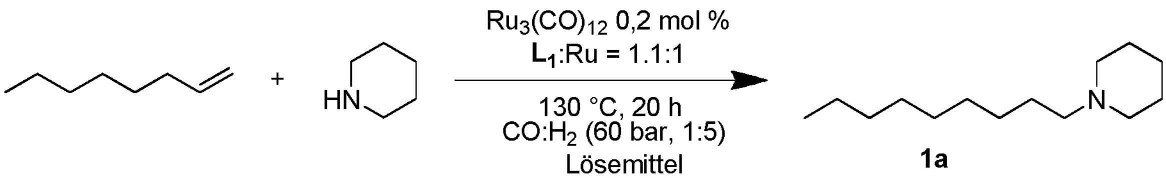


**1a**

Als Katalysator diente $[Ru_3(CO)_{12}]$ mit dem Liganden $\mathbf{L_1}$ unter Verwendung der Lösungsmittel gemäß Tabelle 1. Reaktionsbedingungen: 20 mmol 1-Okten, 24 mmol Piperidin, 0,2 mol % $[Ru_3(CO)_{12}]$, 0,66 mol % $\mathbf{L_1}$, 20 mL Lösungsmittel, 10 bar CO, 50 bar $H_2$, 130 °C, 20 h.
Lösungsmittel sowie Ergebnisse zum Umsetzung, Selektivität und Regioselektivität (n:iso) sind der folgenden Tabelle 1 entnehmbar.

Tabelle 1

| Eintrag | Lösungsmittel | Umsatz[b] [%] | Selektivität [%][b] Amin | Lineares Amin | *N*-formyl Piperidin | n:iso[b] |
|---|---|---|---|---|---|---|
| 1 | Propylencarbonat (PC) | 90 | 44 | 42 | 1 | 96:4 |
| 2 | N-Methyl-2-pyrrolidon (NMP) | 98 | 61 | 57 | <1 | 94:6 |
| 3 | Tetrahydrofuran (THF) | 99 | 84 | 78 | 1 | 93:7 |
| 4 | Ethanol (EtOH) | 99 | 83 | 77 | <1 | 93:7 |
| 5 | Methanol (MeOH) | 99 | 84 | 79 | 1 | 94:6 |
| 6 | Toluol (Tol) | 99 | 85 | 80 | <1 | 94:6 |
| 7[c] | Methanol: Toluol (MeOH:Tol) | >99 | 93 | 88 | <1 | 95:5 |

[b] Bestimmt mittels GC Analyse mit Isooktan als internem Standard.
[c] 10mL MeOH and 10 mL Toluol.

<u>*Beispiel 1 (Tabelle 1)*</u>: Ein 100-mL Autoklav wird befüllt mit $Ru_3(CO)_{12}$ (25,6 mg; 40,0 μmol) und 2 -(Dicyclohexylphosphino)-1-(2-methoxyphenyl)-1 H-imidazol (48,4 mg; 132 μmol). Propylencarbo-nat (20 mL), 1-Okten (3,1 mL; 2,2 g; 20 mmol) und Piperidin (2,4 mL; 2,1 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 90%. Die Selektivität vom C9-Amin beträgt 44% mit einem *n*/*i*-Verhältnis von 96:4. Die Selektivität vom linearen C9-Amin beträgt 42%. Außerdem wird 1% an *N*-Formylpiperidin gefunden.

<u>*Beispiel 2 (Tabelle 1):*</u> Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). *N*-Methyl-2-pyrrolidon (20 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 98%. Die Selektivität vom C9-Amin beträgt 61% mit einem *n*/*i*-Verhältnis von 94:6. Die Selektivität vom linearen C9-Amin beträgt 57%. Außerdem werden weniger als 1% an *N*-Formylpiperidin gefunden.

<u>*Beispiel 3 (Tabelle 1):*</u> Ein 100-mL Autoklav wird befüllt mit $Ru_3(CO)_{12}$ (25,6 mg; 40,0 μmol) und 2 -(Dicyclohexylphosphino)-1-(2-methoxyphenyl)-1 H-imidazol (48,4 mg; 132 μmol). Tetrahydrofuran (20 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 84% mit einem *n*/*i*-Verhältnis von 93:7. Die Selektivität vom linearen C9-Amin beträgt 78%. Außerdem wird 1% an *N*-Formylpiperidin gefunden.

<u>*Beispiele 4 (Tabelle 1):*</u> Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). Ethanol (20 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 83% mit einem *n*/*i*-Verhältnis von 93:7. Die Selektivität vom linearen C9-Amin beträgt 77%. Außerdem werden weniger als 1% an *N*-Formylpiperidin gefunden.

*Beispiel 5 (Tabelle 1):* Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). Methanol (20 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 84% mit einem *n*/*i*-Verhältnis von 94:6. Die Selektivität vom linearen C9-Amin beträgt 79%. Außerdem wird 1% an *N*-Formylpiperidin gefunden.

*Beispiel 6 (Tabelle 1):* Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). Toluol (20 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 85% mit einem *n*/*i*-Verhältnis von 94:6. Die Selektivität vom linearen C9-Amin beträgt 80%. Außerdem werden weniger als 1% an *N*-Formylpiperidin gefunden.

*Beispiel 7 (Tabelle 1):* Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt mehr als 99%. Die Selektivität vom C9-Amin beträgt 93% mit einem *n*/*i*-Verhältnis von 95:5. Die Selektivität vom linearen C9-Amin beträgt 88%. Außerdem werden weniger als 1% an *N*-Formylpiperidin gefunden.

**Erfindungsgemäße Beispiele 8 bis 17**

Ruthenium katalysierte Hydroaminomethylierung von 1-Octen mit Piperidin nach folgendem Reaktionsschema:

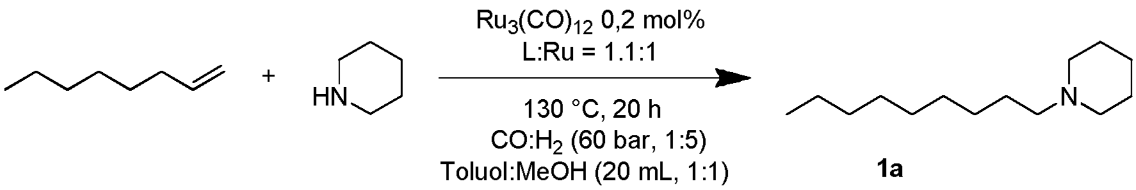


**1a**

Als Katalysator diente $Ru_3(CO)_{12}$ kombiniert mit den Liganden L1 bis L9:

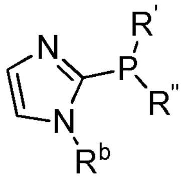

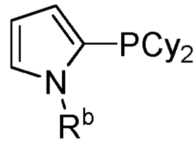

**$L_1$** $R^b$ = 2-OMe($C_6H_4$); R',R" = Cy

**$L_2$** $R^b$ = Me; R',R" = Cy

**$L_3$** $R^b$ = $C_6H_5$; R',R" = Cy

**$L_4$** $R^b$ = 2-OMe($C_6H_4$); R',R" = 'Pr

**$L_5$** $R^b$ = 2-OMe($C_6H_4$); R',R" = Ph

**$L_6$** $R^b$ = 2,4,6-$Me_3$($C_6H_2$); R',R" = Cy

**$L_7$** $R^b$ = $C_6H_5$

**$L_8$** $R^b$ = 2-OMe($C_6H_4$)

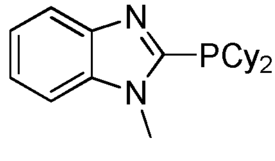

**$L_9$**

Als nicht erfindungsgemäßer Vergleichsligand wurde der Ligand L10 eingesetzt:

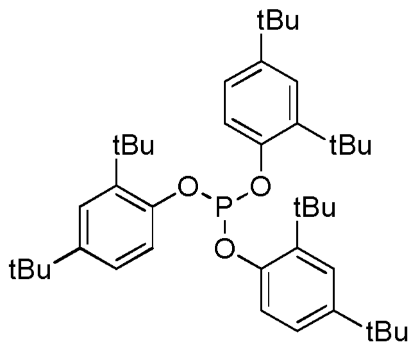

**L10**

Reaktionsbedingungen: 20 mmol 1-Octen, 24 mmol Piperidin, 0.2 mol % $Ru_3(CO)_{12}$, 0.66 mol % Ligand, 10 mL MeOH, 10 mL Toluol, 10 bar CO, 50 bar $H_2$, 130 °C, 20 h.
Umsetzung, Selektivität und Regioselektivität (n:iso) sind der folgenden Tabelle 2 entnehmbar.

Tabelle 2

| Eintrag | Ligand | Umsatz[b] [%] | Selektivität [%][b] Amin | Lineares Amin | *N*-formyl Piperidin | n:iso[b] |
|---|---|---|---|---|---|---|
| 8 | $L_1$ | >99 | 93 | 88 | <1 | 95:5 |
| 9 | $L_2$ | >99 | 90 | 85 | <1 | 94:6 |
| 10 | $L_3$ | >99 | 90 | 86 | 1 | 95:5 |
| 11 | $L_4$ | 99 | 91 | 86 | <1 | 94:6 |
| 12 | $L_5$ | 98 | 69 | 65 | <1 | 94:6 |
| 13 | $L_6$ | >99 | 74 | 71 | 1 | 96:4 |
| 14 | $L_7$ | 83 | 35 | 29 | 2 | 83:17 |
| 15 | $L_8$ | 80 | 34 | 29 | 2 | 85:15 |
| 16 | $L_9$ | 98 | 64 | 61 | 1 | 94:6 |
| 17 | $L_{10}$ | 97 | 30 | 24 | 3 | 79:21 |

[b] Bestimmt mittels GC Analyse mit Isooktan als internem Standard

Wie der Tabelle 2 entnommen werden kann weisen die erfindungsgemäßen Liganden $L_1$ bis $L_9$ bezüglich Aminen und linearen Aminen eine höhere Selektivität auf als der Vergleichsligand $L_{10}$. Des Weiteren weisen die erfindungsgemäßen Liganden $L_1$ bis $L_9$ ein besseres n/iso-Verhältnis auf als der Vergleichsligand $L_{10}$. Bei den Experimenten mit den erfindungsgemäßen Liganden $L_1$ bis $L_9$ wurde weniger an *N*-Formylpiperidin gefunden als bei dem Versuch mit dem Vergleichsligand $L_{10}$.

*Beispiel 8 (Tabelle 2):* Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt mehr als 99%. Die Selektivität vom C9-Amin beträgt 93% mit einem *n*/*i*-Verhältnis von 95:5. Die Selektivität vom linearen C9-Amin beträgt 88%. Außerdem werden weniger als 1% an *N*-Formylpiperidin gefunden.

*Beispiel 9 (Tabelle 2):* Ein 100-mL Autoklav wird befüllt mit $Ru_3(CO)_{12}$ (25,6 mg; 40,0 μmol) und 2 -(Dicyclohexylphosphino)-1-methyl-1 H-imidazol (36,6 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0

mL; 1,4 g; 12 mmol) wird die Reaktionsmischung gaschromatographisch analysiert. Der Umsatz von 1-Okten beträgt mehr als 99%. Die Selektivität vom C9-Amin beträgt 90% mit einem *n*/*i*-Verhältnis von 94:6. Die Selektivität vom linearen C9-Amin beträgt 85%. Außerdem werden weniger als 1% an *N*-Formylpiperidin gefunden.

*Beispiel 10 (Tabelle 2):* Ein 100-mL Autoklav wird befüllt mit $Ru_3(CO)_{12}$ (25,6 mg; 40,0 μmol) und 2-(Dicyclohexylphosphino)-1-phenyl-1 H-imidazol (44,9 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt mehr als 99%. Die Selektivität vom C9-Amin beträgt 90% mit einem *n*/*i*-Verhältnis von 95:5. Die Selektivität vom linearen C9-Amin beträgt 86%. Außerdem werden 1% an *N*-Formylpiperidin gefunden.

*Beispiel 11 (Tabelle 2):* Ein 100-mL Autoklav wird befüllt mit $Ru_3(CO)_{12}$ (25,6 mg; 40,0 μmol) und 2-(Diisopropylphosphino)-1-(2-methoxyphenyl)-1H-imidazol (38,3 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 91% mit einem *n*/*i*-Verhältnis von 94:6. Die Selektivität vom linearen C9-Amin beträgt 86%. Außerdem werden weniger als 1% an *N*-Formylpiperidin gefunden.

*Beispiel 12 (Tabelle 2):* Ein 100-mL Autoklav wird befüllt mit $Ru_3(CO)_{12}$ (25,6 mg; 40,0 μmol) und 2-(Diphenylphosphino)-1-(2-methoxyphenyl)-1 H-imidazol (47,3 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 98%. Die Selektivität vom C9-Amin beträgt 69% mit einem *n*/*i*-Verhältnis von 94:6. Die Selektivität vom linearen C9-Amin beträgt 65%. Außerdem werden weniger als 1% an *N*-Formylpiperidin gefunden.

*Beispiel 13 (Tabelle 2):* Ein 100-mL Autoklav wird befüllt mit $Ru_3(CO)_{12}$ (25,6 mg; 40,0 μmol) und 2-(Dicyclohexylphosphino)-1-mesityl-1H-imidazol (50,4 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt mehr als 99%. Die Selektivität vom C9-Amin beträgt 74% mit einem *n*/*i*-Verhältnis von 96:4. Die Selektivität vom linearen C9-Amin beträgt 71%. Außerdem werden 1% an *N*-Formylpiperidin gefunden.

*Beispiel 14 (Tabelle 2):* Ein 100-mL Autoklav wird befüllt mit $Ru_3(CO)_{12}$ (25,6 mg; 40,0 μmol) und 2-(Dicyclohexylphosphino)-1-phenyl-1H-pyrrol (44,7 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 83%. Die Selektivität vom C9-Amin beträgt 35% mit einem *n*/*i*-Verhältnis von 83:17. Die Selektivität vom linearen C9-Amin beträgt 29%. Außerdem werden 2% an *N*-Formylpiperidin gefunden.

*Beispiel 15 (Tabelle 2):* Ein 100-mL Autoklav wird befüllt mit $Ru_3(CO)_{12}$ (25,6 mg; 40,0 μmol) und 2-(Dicyclohexylphosphino)-1-(2-methoxyphenyl)-1 H-pyrrol (48,7 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 80%. Die Selektivität vom C9-Amin beträgt 34% mit einem *n*/*i*-Verhältnis von 85:15. Die Selektivität vom linearen C9-Amin beträgt 29%. Außerdem werden 2% an *N*-Formylpiperidin gefunden.

*Beispiel 16 (Tabelle 2):* Ein 100-mL Autoklav wird befüllt mit $Ru_3(CO)_{12}$ (25,6 mg; 40,0 μmol) und 2-(Dicyclohexylphosphino)-1-methyl-1 H-benzo[d]imidazol (43,3mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 98%. Die Selektivität vom C9-Amin beträgt 64% mit einem *n*/*i*-Verhältnis von 94:6. Die Selektivität vom linearen C9-Amin beträgt 61%. Außerdem werden 1% an *N*-Formylpiperidin gefunden.

*Beispiel 17 (Tabelle 2):* Ein 100-mL Autoklav wird befüllt mit $Ru_3(CO)_{12}$ (25,6 mg; 40,0 μmol) und Tris(2,4-di-tert-butylphenyl) phosphit (85,3mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 97%. Die Selektivität vom C9-Amin beträgt 30% mit einem *n*/*i*-Verhältnis von 79:21. Die Selektivität vom linearen C9-Amin beträgt 24%. Außerdem werden 3% an *N*-Formylpiperidin gefunden.

**Erfindungsgemäße Beispiele 18 bis 22**

Ruthenium-katalysierte Hydroaminomethylierung von 1-Octen mit Piperidin nach folgendem Reaktionsschema unter Bestimmung der Aktivitäten (TOF) unter Einsatz der Liganden $\mathbf{L_1}$-$\mathbf{L_5}$: Reaktionsbedingungen: 20 mmol 1-Okten, 24 mmol Piperidin, 0.1 mol % $Ru_3(CO)_{12}$, 0.33 mol % Ligand, 10 mL MeOH, 10 mL Toluol, 10 bar CO, 50 bar $H_2$, 130 °C, 0.5 h

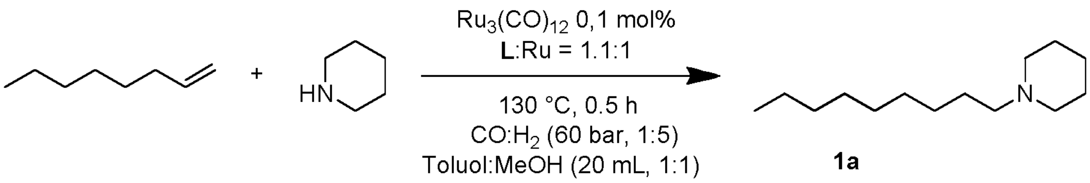


**1a**

Tabelle 3

| Eintrag | Ligand | Umsatz [%][b] | Selektivität [%][b] | | *n:isob* | TOF [mol*mol $Ru^{-1}$*$h^{-1}$] |
|---|---|---|---|---|---|---|
| | | | Amin | Lineares Amin | | |
| 18 | $\mathbf{L_1}$ | 78 | 85 | 82 | 97:3 | 440 |
| 19 | $\mathbf{L_2}$ | 56 | 86 | 83 | 96:4 | 320 |
| 20 | $\mathbf{L_3}$ | 75 | 76 | 73 | 96:4 | 380 |
| 21 | $\mathbf{L_4}$ | 72 | 83 | 81 | 97:3 | 400 |
| 22 | $\mathbf{L_5}$ | 81 | 70 | 67 | 95:5 | 380 |

[b] Bestimmt mittels GC Analyse mit Isooctan als internem Standard.

Wie der Tabelle 3 entnommen werden kann, zeichnen sich die erfindungsgemäßen Liganden $L_1$-$L_5$ in der Ruthenium-katalysierten Hydroaminomethylierung von 1-Octen mit Piperidin durch sehr gute TOF's von 320 und mehr und sehr gute Regioselektivitäten aus. Die Aufgabe wurde durch den Einsatz dieser Liganden also erfüllt.

*Beispiel 18 (Tabelle 3):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 0.5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 78%. Die Selektivität vom C9-Amin beträgt 85% mit einem *n*/*i*-Verhältnis von 97:3. Die Selektivität vom linearen C9-Amin beträgt 82%. Turnover-Frequenz 440 mol*mol $Ru^{-1}$*$h^{-1}$.

*Beispiel 19 (Tabelle 3):* Ein 100-mL Autoklav wird befüllt mit $Ru_3(CO)_{12}$ (12,8 mg; 20,0 μmol) und 2 -(Dicyclohexylphosphino)-1-methyl-1H-imidazol (18,3 mg; 66 μmol), Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 0.5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 56%. Die Selektivität vom C9-Amin beträgt 86% mit einem *n*/*i*-Verhältnis von 96:4. Die Selektivität vom linearen C9-Amin beträgt 83%. Turnover-Frequenz 320 mol*mol $Ru^{-1}*h^{-1}$.

*Beispiel 20 (Tabelle 3):* Ein 100-mL Autoklav wird befüllt mit $Ru_3(CO)_{12}$ (12,8 mg; 20,0 μmol) und 2-(Dicyclohexylphosphino)-1-phenyl-1H-imidazol (22.4 mg; 66 μmol), Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 0.5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 75%. Die Selektivität vom C9-Amin beträgt 76% mit einem *n*/*i*-Verhältnis von 96:4. Die Selektivität vom linearen C9-Amin beträgt 73%. Turnover-Frequenz 380 mol*mol $Ru^{-1}*h^{-1}$.

*Beispiel 21 (Tabelle 3):* Ein 100-mL Autoklav wird befüllt mit $Ru_3(CO)_{12}$ (12,8 mg; 20,0 μmol) und 2-(diisopropylphosphino)-1-(2-methoxyphenyl)-1H-imidazol (19.1 mg; 66 μmol), Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 0.5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 72%. Die Selektivität vom C9-Amin beträgt 83% mit einem *n*/*i*-Verhältnis von 97:3. Die Selektivität vom linearen C9-Amin beträgt 81%. Turnover-Frequenz 400 mol*mol $Ru^{-1}*h^{-1}$.

*Beispiel 22 (Tabelle 3):* Ein 100-mL Autoklav wird befüllt mit $Ru_3(CO)_{12}$ (12,8 mg; 20,0 μmol) und 2-(diphenylphosphino)-1-(2-methoxyphenyl)-1 H-imidazol (23.6 mg; 66 μmol), Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 0.5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 81%. Die Selektivität vom C9-Amin beträgt 70% mit einem *n*/*i*-Verhältnis von 95:5. Die Selektivität vom linearen C9-Amin beträgt 67%. Turnover-Frequenz 380 mol*mol $Ru^{-1}*h^{-1}$.

**Erfindungsgemäße Beispiele 23 bis 29**

Ruthenium katalysierte Hydroaminomethylierung von 1-Octen mit Piperidin nach folgendem Reaktionsschema unter Variation der Reaktionsparameter:

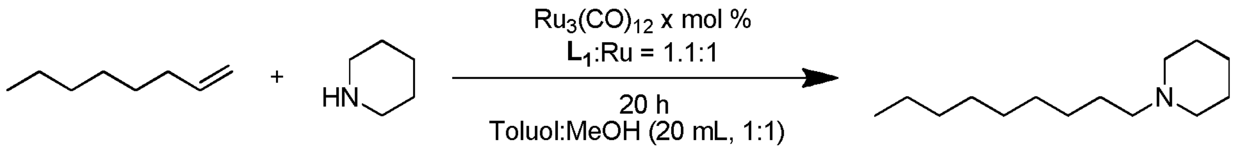


**1a**

Reaktionsbedingungen: 20 mmol 1-Okten, 24 mmol Piperidin, x mol % $Ru_3(CO)_{12}$, 3.3 x mol % $\mathbf{L_1}$, 10 mL MeOH, 10 mL Toluole, 130 °C, 20 h.

Tabelle 4

| Eintrag | x | $CO:H_2$ [bar] | Conversion [%][b] | Selektivität [%][b] Amin | Lineares Amin | *N*-formyl Piperidin | n:iso[b] |
|---|---|---|---|---|---|---|---|
| 23 | 0.2 | 10:50 | >99 | 93 | 88 | <1 | 95:5 |
| 24 | 0.1 | 10:50 | 99 | 89 | 85 | <1 | 95:5 |

(fortgesetzt)

| Eintrag | x | $CO:H_2$ | Conversion | Selektivität [%][b] | | | n:iso[b] |
|---|---|---|---|---|---|---|---|
| | | [bar] | [%][b] | Amin | Lineares Amin | *N*-formyl Piperidin | |
| 25 | 0.05 | 10:50 | 99 | 85 | 81 | 2 | 95:5 |
| 26[c] | 0.1 | 10:50 | 99 | 79 | 76 | <1 | 96:4 |
| 27 | 0.1 | 10:40 | 99 | 84 | 79 | 1 | 94:6 |
| 28 | 0.1 | 7:35 | 99 | 87 | 83 | 1 | 95:5 |
| 29 | 0.1 | 20:40 | 99 | 81 | 77 | 2 | 95:5 |

[b] Bestimmt mittels GC Analys mit Isooktan als internem Standard.
[c] 120 °C.

Wie Tabelle 4 entnommene werden kann ist es möglich, die Hydroaminomethylierung mit nur 0,05 mmol% $[Ru_3(CO)_{12}]$ (Eintrag 5) bei weiterhin sehr guten Ausbeuten des Zielprodukts und sehr guten n/iso-Selektivitäten. Es handelt sich somit um ein sehr aktives und selektives Verfahren. Ferner ist der Gehalt an N-Formypiperidin in allen Fällen sehr gering.

*Beispiel 23 (Tabelle 4):* Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt mehr als 99%. Die Selektivität vom C9-Amin beträgt 93% mit einem *n*/*i*-Verhältnis von 95:5. Die Selektivität vom linearen C9-Amin beträgt 88%. Außerdem werden weniger als 1% an *N*-Formylpiperidin gefunden.

*Beispiel 24 (Tabelle 4):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 89% mit einem *n*/*i*-Verhältnis von 95:5. Die Selektivität vom linearen C9-Amin beträgt 85%. Außerdem werden weniger als 1% an *N*-Formylpiperidin gefunden.

*Beispiel 25 (Tabelle 4):* Ein 100-mL Autoklav wird befüllt mit $Ru_3(CO)_{12}$ (6,4 mg; 10,0 μmol) und 2-(Dicyclohexylphosphino)-1-(2-methoxyphenyl)-1H-imidazol (12,1 mg; 33 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 85% mit einem *n*/*i*-Verhältnis von 95:5. Die Selektivität vom linearen C9-Amin beträgt 81%. Außerdem werden weniger als 2% an *N*-Formylpiperidin gefunden.

*Beispiel 26 (Tabelle 4):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 120 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 79% mit einem *n*/*i*-Verhältnis von 96:4. Die Selektivität vom linearen C9-Amin beträgt 76%. Außerdem werden weniger als 1% an *N*-Formylpiperidin gefunden.

*Beispiele 27 (Tabelle 4):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas

($CO/H_2$ 1:4 5,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 84% mit einem *n*/*i*-Verhältnis von 94:6. Die Selektivität vom linearen C9-Amin beträgt 79%. Außerdem werden 1% an *N*-Formylpiperidin gefunden.

*Beispiel 28 (Tabelle 4):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:5 4,2 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 87% mit einem *n*/*i*-Verhältnis von 95:5. Die Selektivität vom linearen C9-Amin beträgt 83%. Außerdem werden 1% an *N*-Formylpiperidin gefunden.

*Beispiel 29 (Tabelle 4):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Piperidin (2,4 mL; 2,0 g; 24 mmol) werden hinzugegeben und es wird Synthesegas ($CO/H_2$ 1:2 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 99%. Die Selektivität vom C9-Amin beträgt 81% mit einem *n*/*i*-Verhältnis von 95:5. Die Selektivität vom linearen C9-Amin beträgt 77%. Außerdem werden 2% an *N*-Formylpiperidin gefunden.

## Erfindungsgemäße Beispiele 30 bis 45

Ruthenium katalysierte Hydroaminomethylierung von 1-Octen mit verschiedenen Aminen nach folgendem Reaktionsschema:

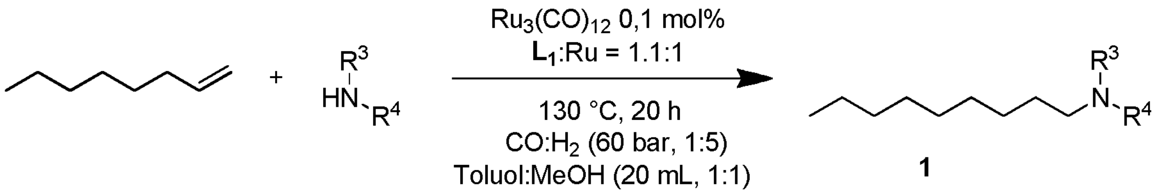


**1**

Reaktionsbedindungen: 20 mmol 1-Octen, 24 mmol Amine, 0.1 mol % $Ru_3(CO)_{12}$, 0.33 mol % $\mathbf{L_1}$, 10 mL MeOH, 10 mL Toluol, 10 bar CO, 50 bar $H_2$, 130 °C, 20 h.

Tabelle 5

| Eintrag | Amin | Hauptprodukt 1 | GC Ausbeute [%][b] | Isolierte Ausbeute [%][c] | n:iso |
|---|---|---|---|---|---|
| 30 | 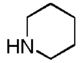 | 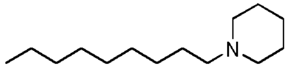 1a | 88 | 81 | 95:5 |
| 31 | 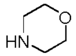 | 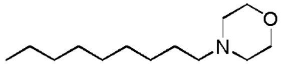 1b | 90 | 85 | 94:6 |
| 32 | 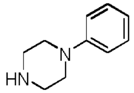 | 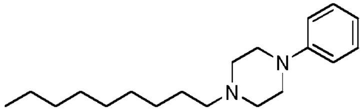 1c | 89 | 80 | 93:7 |
| 33 | 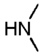 | 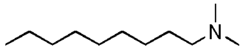 1d | 84 | 74 | 94:6 |
| 34 | 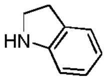 | 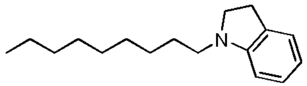 1e | 90 | 77 | 93:7 |
| 35 | 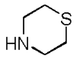 | 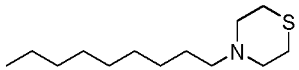 1f | 10 | 8 | 92:8 |
| 36 | 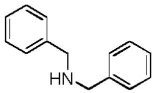 | 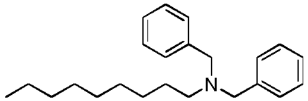 1g | 89 | 83 | 93:7 |
| 37 | 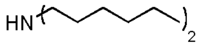 | 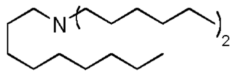 1h | 87 | 61 | 96:4 |
| 38 | 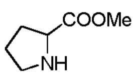 | 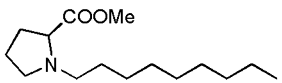 1i | 84 | 79 | 95:5 |
| 39 | 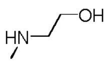 | 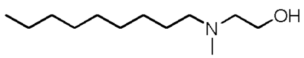 1j | 91 | 73 | 94:6 |

(fortgesetzt)

| Eintrag | Amin | Hauptprodukt 1 | GC Ausbeute [%][b] | Isolierte Ausbeute [%][c] | n:iso |
|---|---|---|---|---|---|
| 40 | 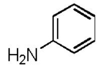 | 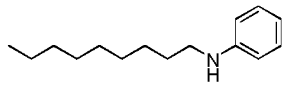 1k | 76 | 68 | 94:6 |
| 41 | 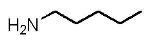 | 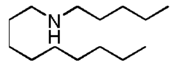 1l | 71 | 62 | 93:7 |
| 42 | 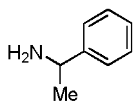 | 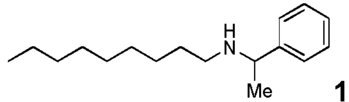 1m | 82 | 77 | 93:7 |
| 43 | 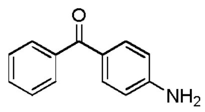 | 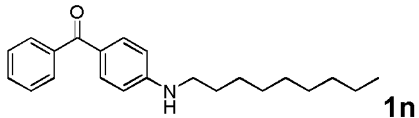 1n | 47 | 36 | 96:4 |
| 44 | 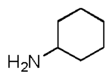 | 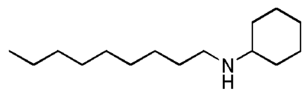 1o | 83 | 76 | 94:6 |
| 45[d] | 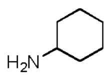 | 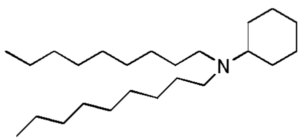 1p | 70 | 65 | 87:13 |

[b] Bestimmt mittels GC Analyse mit Isooktan als internem Standard.
[c] isolierte Ausbeute nach Destillation oder Säulenchromatographie.
[d] 20 mmol Amin, 40 mmol Olefin, 0.2 mol % $Ru_3(CO)_{12}$, 0.66 mol % Ligand, 20 bar CO, 40 bar $H_2$.

Tabelle 5 kann entnommen werden, dass eine Vielzahl von Aminen in der Ruthenium-katalysierten Hydroaminomethylierung eingesetzt werden können. Es ist möglich, primäre und sekundäre, alkyl-, cyclisch-, heterocyclisch- und aromatisch-substitiierte Amine zu verwenden, wobei auch funktionelle Gruppen toleriert werden. Alle zeichnen sich durch gute bis sehr gute Ausbeuten und gute n/iso-Selektivitäten aus. Es wurde völlig überraschend gefunden, dass sogar Thiomorpholin umgesetzt werden konnte (Eintrag 35). Durch die in Tabelle 5 beschriebenen Bespiele konnte gezeigt werden, dass das entwickelte Verfahren universell anwendbar ist.

*Beispiel 30 (Tabelle 5):* Entspricht Beispiel 24. Nach der Zugabe des internen Standards (Isooktan, 2,0 mL; 1,4 g; 12 mmol) wird die Reaktionsmischung gaschromatographisch analysiert. Die Ausbeute an 1-Nonylpiperidin beträgt 88% mit einem *n*/*i*-Verhältnis von 95:5. 1-n-Nonylpiperidin wurde nach Kugelrohr-Destillation (Siedepunkt 110-115 °C bei 3 mbar) in 81% Ausbeute und 97% Reinheit erhalten.

*Beispiel 31 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Morpholin (2,1 mL; 2,1 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 4-nonylmorpholine beträgt 90% mit einem *n*/*i*-Verhältnis von 94:6. 4-Nonylmorpholine wurde anschließender Kugelrohr-Destillation (Siedepunkt 120-125 °C bei 1,2 mbar) in 85% Ausbeute und 96% Reinheit erhalten.

*Beispiel 32 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und 1-Phenylpiperazin (3,7 mL; 3,9 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-Nonyl-4-Phenylpiperazin beträgt 89% mit einem *n*/*i*-Verhältnis von 93:7. 1-Nonyl-4-Phenylpiperazin gewonnen durch Säulenchromatographie an Silicagel (Eluent, Petroleumether: Ethylacetat 10:1), Schmelzpunkt 68°C wurde in 80% Ausbeute und 96% Reinheit erhalten.

*Beispiel* 33 *(Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Dimethylamine 2M in Methanol (12 mL; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an *N, N*-Dimethylnonan-1-amin beträgt 84% mit einem *n*/*i*-Verhältnis von 94:6. *N,N*-Dimethylnonan-1-amin wurde nach anschließender Kugelrohr-Destillation (Siedepunkt 60-65 °C bei 1,2 mbar) in 74% Ausbeute und 98% Reinheit erhalten.

*Beispiel 34 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Indolin (2,7 mL; 2,8 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-Nonylindolin beträgt 90% mit einem *n*/*i*-Verhältnis von 93:7. 1-Nonylindolin wurde nach anschließender Kugelrohr-Destillation (Siedepunkt 170-175 °C bei 1,2 mbar) in 77% Ausbeute und 94% Reinheit erhalten.

*Beispiel 35 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Thiomorpholin (2,4 mL; 2,5 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 4-Nonylthiomorpholin beträgt 10% mit einem *n*/*i*-Verhältnis von 92:8. 4-Nonylthiomorpholin wurde nach anschließender Kugelrohr-Destillation (Siedepunkt 130-135 °C bei 1,2 mbar) in 8% Ausbeute und 92% Reinheit

erhalten.

*Beispiel* 36 *(Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Dibenzylamin (4,6 mL; 4,7 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an *N,N*-Dibenzylnonan-1-amin beträgt 89% mit einem *n*/*i*-Verhältnis von 93:7. *N,N*-Dibenzylnonan-1-amin wurde isoliert mittels Säulenchromatographie an Silicagel (Eluent, Petroleumether: Ethylacetat 10:1). Bp 210°C, 1 mbar. 98% Reinheit in 83% Ausbeute

*Beispiel 37 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Dihexylamin (5,6 mL; 4,4 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an *N,N*-Dihexylnonan-1-amin beträgt 87% mit einem *n*/*i*-Verhältnis von 96:4. *N,N*-Dihexylnonan-1-amin wurde nach Kugelrohr-Destillation (Siedepunkt 150-155 °C bei 2 mbar) in 61% Ausbeute und 95% Reinheit erhalten.

*Beispiel* 38 *(Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Methylpyrrolidin-2-carboxylat (3,1 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an Methyl-1-nonylpyrrolidin-2-carboxylat beträgt 84% mit einem *n*/*i*-Verhältnis von 95:5. Methyl-1-nonylpyrrolidin-2-carboxylat wurde nach Kugelrohr-Destillation (Siedepunkt 150-155 °C bei 1,5 mbar) in 79% Ausbeute und 97% Reinheit erhalten.

*Beispiel* 39 *(Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und *N*-Methyl-aminoethanol (1,9 mL; 1,8 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 2-(Methyl(nonyl)amino)ethanol beträgt 91% mit einem *n*/*i*-Verhältnis von 94:6. 2-(Methyl(nonyl)amino)ethanol wurde nach Kugelrohr-Destillation (Siedepunkt 135-140 °C bei 1,2 mbar) in 73% Ausbeute und 94% Reinheit erhalten.

*Beispiel 40 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Anilin (2,2 mL; 2,2 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an *N*-nonylanilin beträgt 76% mit einem *n*/*i*-Verhältnis von 94:6. *N*-Nonylanilin wurde nach Kugelrohr-Destillation (Siedepunkt 140-145 °C bei 1,2 mbar) in 68% Ausbeute und 93% Reinheit erhalten.

*Beispiel 41 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (1 mL), Toluol (10 mL), 1-Okten (3,1 mL; 2,2 g; 20 mmol) und Amylamin (2,8 mL; 2,1 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an *N*-pentylnonan-1-amin beträgt 71% mit einem *n*/*i*-Verhältnis von 93:7. *N*-Pentylnonan-1-amin wurde nach Kugelrohr-Destillation (Siedepunkt 140-145 °C bei 1,5 mbar) in 62% Ausbeute und 94% Reinheit erhalten.

*Beispiel 42 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und 1-Phenylethanamin (3,1 mL; 2,9 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an *N*-(1-Phenylethyl)nonan-1-amin beträgt 82% mit einem *n*/*i*-Verhältnis von 93:7. *N*-(1-Phenylethyl)nonan-1-amin wurde nach Kugelrohr-Destillation (Siedepunkt 160-165 °C bei 1,2 mbar) in 77% Ausbeute und 92% Reinheit erhalten.

*Beispiel 43 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und (4-Aminophenyl)(phenyl)methanon (4,7 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an (4-(Nonylamino)phenyl)(phenyl)methanon beträgt 47% mit einem *n*/*i*-Verhältnis von 96:4. (4-(Nonylamino)phenyl)(phenyl)methanon als leicht gelber Feststoff, isoliert durch Säulenchromatographie an Silicagel (Eluent, Petroleumether: Ethylacetat 5:1) Schmelzpunkt 30°C in 36% Ausbeute und 98% Reinheit erhalten.

*Beispiel 44 (Tabelle 5):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Okten (3,1 mL; 2,2 g; 20 mmol) und Cyclohexanamin (2,8 mL; 2,4 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an *N*-Nonylcyclohexanamin beträgt 83% mit einem *n*/*i*-Verhältnis von 94:6. *N*-Nonylcyclohexanamin wurde nach Kugelrohr-Destillation (Siedepunkt 140-145 °C bei 1,2 mbar) in 76% Ausbeute und 99% Reinheit erhalten.

*Beispiel 45 (Tabelle 5):* Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). Methanol (10 mL), Toluol (10 mL), 1-Okten (6,2 mL; 4,4 g; 40 mmol) und Cyclohexanamin (2,3 mL; 2,0 g; 20 mmol) werden hinzugegeben und es wird Synthesegas ($CO/H_2$ 1:2 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an *N,N*-dinonylcyclohexanamin beträgt 70% mit einem *n*/*i*-Verhältnis von 87:13. *N,N*-dinonylcyclohexanamin wurde nach Kugelrohr-Destillation (Siedepunkt 200 °C bei 1,8 mbar) in 65% Ausbeute und 93% Reinheit erhalten.

**Erfindungsgemäße Beispiele 46 bis 60**

Ruthenium katalysierte Hydroaminomethylierung von Piperidin mit verschiedenen Alkenen nach folgendem Reaktionsschema:

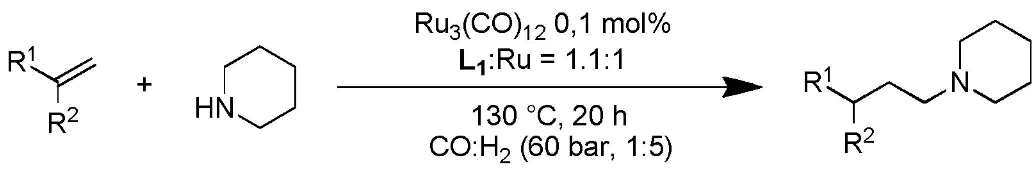


2

Reaktionsbedingungen: 20 mmol Alken, 24 mmol Piperidin, 0.1 mol % $Ru_3(CO)_{12}$, 0.33 mol % $\mathbf{L_1}$, 10 mL MeOH, 10 mL Toluol, 10 bar CO, 50 bar $H_2$, 130 °C, 20 h.

Tabelle 6

| Eintrag | Alken | Hauptprodukt **2** | GC Ausbeute [%][b] | Isolierte Ausbeute [%][c] | n:iso |
|---|---|---|---|---|---|
| 46 | 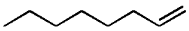 | 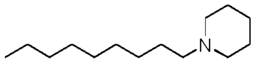 **1a** | 88 | 81 | 95:5 |
| 47 | 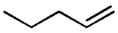 | 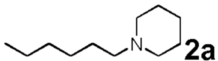 **2a** | 82 | 80 | 93:7 |
| 48 | 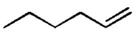 | 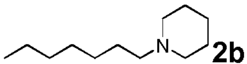 **2b** | 83 | 80 | 95:5 |
| 49 | 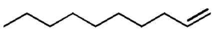 | 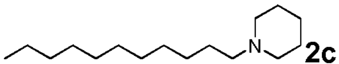 **2c** | 85 | 80 | 93:7 |
| 50[d] | 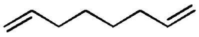 | 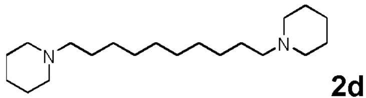 **2d** | 79 | 70 | 89:11 |
| 51[e] | 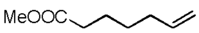 | 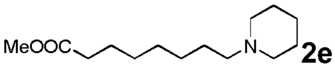 **2e** | 78 | 67 | 95:5 |
| 52 | 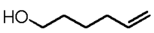 | 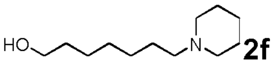 **2f** | 91 | 85 | 95:5 |
| 53 | 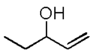 | 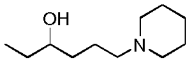 **2g** | 84 | 76 | 99:1 |
| 54 | 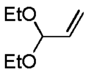 | 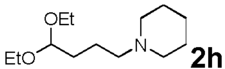 **2h** | 75 | 64 | 86:14 |
| 55 | 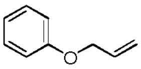 | 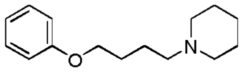 **2i** | 34 | 30 | 98:2 |
| 56[f] | 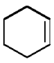 | 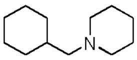 **2j** | 65 | 58 | - |
| 57[g] | 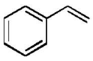 | 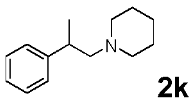 **2k** | 96 | 80 | 45:55 |
| 58 | 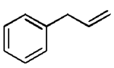 | 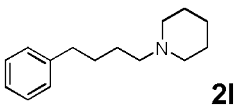 **2l** | 90 | 84 | 88:12 |
| 59[h] | 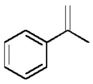 | 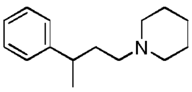 **2m** | 64 | 55 | >99:1 |

(fortgesetzt)

| Eintrag | Alken | Hauptprodukt **2** | GC Ausbeute [%][b] | Isolierte Ausbeute [%][c] | n:iso |
|---|---|---|---|---|---|
| 60 | 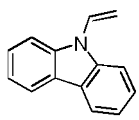 | 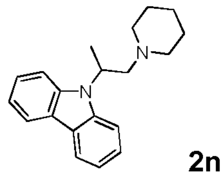 **2n** | 53 | 45 | 41:59 |

[b] Selektivität wurde durch GC Analyse mit Isooktan als internem Standard bestimmt.
[c] Isolierte Ausbeute nach Destillation oder Säulenchromatographie.
[d] 20 mmol Alken, 48 mmol Piperidin, 0.2 mol % $Ru_3(CO)_{12}$, 0.66 mol % Ligand, 20 bar CO, 40 bar $H_2$.
[e] Reaktion lief mit 7 mmol Alken. [f]160 °C. [g]5 h. [h]40 h.

Wie Tabelle 6 entnommen werden kann, wurden verschiedene Alkene mit Piperidin in der Ruthenium-katalysierten Hydroaminomethylierung umgesetzt. Es konnte gezeigt werden, dass alkyl-, cyclisch-, heterocyclisch- und aromatisch- substituierte Alkene in guten Ausbeuten hydroaminomethyliert werden konnten. Auch mehrfach ungesättigte Alkene sowie funktionelle Gruppen werden unter den Reaktionsbedingungen toleriert.

*Beispiel 46 (Tabelle 6):* Entspricht Beispiel 24. Nach der Zugabe des internen Standards (Isooktan, 2,0 mL; 1,4 g; 12 mmol) wird die Reaktionsmischung gaschromatographisch analysiert. Die Ausbeute an 1-Nonylpiperidin beträgt 88% mit einem *n*/*i*-Verhältnis von 95:5. 1-Nonylpiperidin wurde nach Kugelrohr-Destillation (Siedepunkt 110-115 °C bei 3 mbar) in 81% Ausbeute und 97% Reinheit erhalten.

*Beispiel 47 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Penten (2,2 mL; 1,4 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-Hexylpiperidin beträgt 82% mit einem *n*/*i*-Verhältnis von 93:7. 1-Hexylpiperidin wurde nach Kugelrohr-Destillation (Siedepunkt 90-95 °C bei 3 mbar) in 80% Ausbeute und 96% Reinheit erhalten.

*Beispiel 48 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Hexen (2,5 mL; 1,7 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-Heptylpiperidin beträgt 83% mit einem *n*/*i*-Verhältnis von 95:5. 1-Heptylpiperidin wurde nach Kugelrohr-Destillation (Siedepunkt 80-85 °C bei 1 mbar) in 80% Ausbeute und 98% Reinheit erhalten.

*Beispiel 49 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Decen (3,8 mL; 2,8 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-Undecylpiperidin beträgt 85% mit einem *n*/*i*-Verhältnis von 93:7. 1-Undecylpiperidin wurde nach Kugelrohr-Destillation (Siedepunkt 140-145 °C bei 3 mbar) in 80% Ausbeute und 95% Reinheit erhalten.

*Beispiel 50 (Tabelle 6):* Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). Methanol (10 mL), Toluol (10 mL), 1,7-Octadien (3,0 mL; 2,2 g; 20 mmol) und Piperidin (4,8 mL; 4,0 g; 48 mmol) werden hinzugegeben und es wird Synthesegas ($CO/H_2$ 1:2 6,0 MPa) aufgepresst. Die Reaktionsmischung wird auf 130 °C erwärmt und 20 Stunden bei dieser

Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1,10-Di(piperidin-1-yl)decan beträgt 79% mit einem *n*/*i*-Verhältnis von 89:11. 1,10-Di(piperidin-1-yl)decan wurde nach Kugelrohr-Destillation (Siedepunkt 180-185 °C bei 1,2 mbar) in 70% Ausbeute und 88% Reinheit erhalten.

*Beispiel 51 (Tabelle 6):* Ein 100-mL Autoklav wird befüllt mit $Ru_3(CO)_{12}$ (4,5 mg; 7,0 μmol) und 2-(Dicyclohexylphosphino)-1-(2-methoxyphenyl)-1H-imidazol (8,5 mg; 23 μmol). Methanol (10 mL), Toluol (10 mL), Methyl 6-heptenoat (1,0 g; 7 mmol) und Piperidin (0,8 mL; 0,7 g; 8,4 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an Methyl 8-(piperidin-1-yl)octanoat beträgt 78% mit einem *n*/*i*-Verhältnis von 95:5. Methyl 8-(piperidin-1-yl)octanoat wurde nach Kugelrohr-Destillation (Siedepunkt 170-175 °C bei 4 mbar) in 67% Ausbeute und 99% Reinheit erhalten.

*Beispiel 52 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 5-Hexen-1-ol (2,4 mL; 2,0 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 7-(Piperidin-1-yl)heptan-1-ol beträgt 91% mit einem *n*/*i*-Verhältnis von 95:5. 7-(Piperidin-1-yl)heptan-1-ol wurde nach Kugelrohr-Destillation (Siedepunkt 160-165 °C bei 8 mbar) in 85% Ausbeute und 97% Reinheit erhalten.

*Beispiel 53 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 1-Penten-3-ol (2,1 mL; 1,7 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 6-(Piperidin-1-yl)hexan-3-ol beträgt 84% mit einem *n*/*i*-Verhältnis von 99:1. 6-(Piperidin-1-yl)hexan-3-ol wurde nach Kugelrohr-Destillation (Siedepunkt 100-105 °C bei 1,2 mbar) in 76% Ausbeute und 98% Reinheit erhalten.

*Beispiel 54 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 3,3-Diethoxyprop-1-en (2,4 mL; 2,0 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-(4,4-Diethoxybutyl)piperidin beträgt 75% mit einem *n*/*i*-Verhältnis von 86:14. 1-(4,4-Diethoxybutyl)piperidin wurde nach Kugelrohr-Destillation (Siedepunkt 115-120 °C bei 1,2 mbar) in 64% Ausbeute und 86% Reinheit erhalten.

*Beispiel 55 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10mL), Toluol (10 mL), Allyloxybenzol (2,7 mL; 2,6 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-(4-Phenoxybutyl)piperidin beträgt 34% mit einem *n*/*i*-Verhältnis von 98:2. 1-(4-Phenoxybutyl)piperidin wurde nach Kugelrohr-Destillation (Siedepunkt 160-165 °C bei 1,2 mbar) in 30% Ausbeute und 99% Reinheit erhalten.

*Beispiel 56 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), Cyclohexen

(2,0 mL; 1,6 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 160 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-(Cyclohexylmethyl)piperidin beträgt 65%. 1-(Cyclohexylmethyl)piperidin wurde nach Kugelrohr-Destillation (Siedepunkt 90-95 °C bei 1,2 mbar) in 58% Ausbeute und 99% Reinheit erhalten.

*Beispiele 57 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), Styrol(2,3 mL; 2,1 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 5 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-(2-Phenylpropyl)piperidin beträgt 96% mit einem *n*/*i*-Verhältnis von 45:55.. 1-(2-Phenylpropyl)piperidin wurde isoliert durch Säulenchromatographie an Silicagel (Eluent, Petroleumether: Ethylacetat 4:1) in 80% Ausbeute, Siedepunkt 120°C, 1.0 m bar.

*Beispiel 58 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), Allylbenzol (2,6 mL; 2,4 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-(4-Phenylbutyl)piperidin beträgt 90% mit einem *n*/*i*-Verhältnis von 88.12. 1-(4-Phenylbutyl)piperidin wurde nach Kugelrohr-Destillation (Siedepunkt 130-135 °C bei 1,2 mbar) in 84% Ausbeute und 99% Reinheit erhalten.

*Beispiel 59 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), Prop-1-en-2-ylbenzol (2,6 mL; 2,4 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 1-(3-Phenylbutyl)piperidin beträgt 64% mit einem *n*/*i*-Verhältnis von 99:1. 1-(3-Phenylbutyl)piperidin wurde nach Kugelrohr-Destillation (Siedepunkt 130-135 °C bei 1,2 mbar) in 55% Ausbeute und 99% Reinheit erhalten.

*Beispiel 60 (Tabelle 6):* Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 9-Vinyl-9H-carbazol (3,9 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Die Ausbeute an 9-(1-(Piperidin-1-yl)propan-2-yl)-9H-carbazol beträgt 53% mit einem *n*/*i*-Verhältnis von 41:59. 9-(1-(Piperidin-1-yl)propan-2-yl)-9H-carbazol wurde isoliert durch Säulenchromatographie an Silicagel (Eluent, Petroleumether: Ethylacetat 2:1) in 45% Ausbeute, Siedepunkt 220°C, 1.5 m bar.

**Erfindungsgemäße Beispiele 61 bis 63**

Hydroaminomethylierung von 2-Octen mit Piperidin

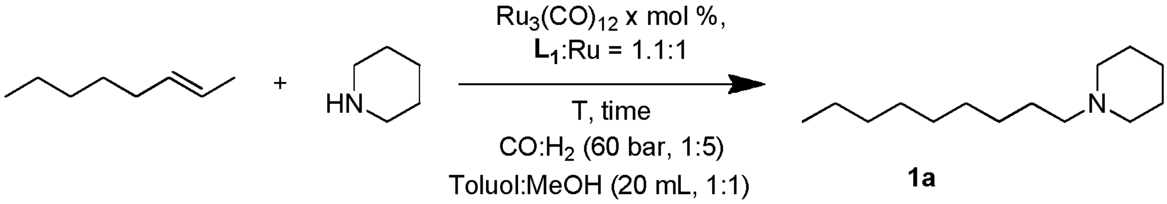

1a

Reaktionsbedingungen: 20 mmol 2-Octen, 24 mmol Piperidin, x mol % $Ru_3(CO)_{12}$, 3.3 x mol % $\mathbf{L_1}$, 10 mL MeOH, 10 mL Toluol, 10 bar CO, 50 bar $H_2$.

Tabelle 7

| Eintrag | x | T [°C] | Zeit | Umsatz [%] | Selektivität [%][b] Amin | Lineares Amin | *N*-formyl Piperidin | n:iso[b] |
|---|---|---|---|---|---|---|---|---|
| 61 | 0.1 | 130 | 20 | 40 | 70 | 50 | 2 | 71:29 |
| 62 | 0.1 | 160 | 40 | 89 | 60 | 49 | 9 | 81:19 |
| 63 | 0.2 | 160 | 30 | 94 | 71 | 59 | 10 | 83:17 |

[b] Bestimmt durch GC Analyse mit Isooctane als internem Standard.

*Beispiel 61 (Tabelle 7)*: Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 2-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 130 °C erwärmt und 20 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 40%. Die Selektivität vom C9-Amin beträgt 70% mit einem *n*/*i*-Verhältnis von 71.29. Die Selektivität vom linearen C9-Amin beträgt 50%. Außerdem werden 2% an *N*-Formylpiperidin gefunden.

*Beispiel 62 (Tabelle 7)*: Ein 100-mL Autoklav wird befüllt mit $Ru_3(CO)_{12}$ (12,8 mg; 20,0 μmol) und 2-(Dicyclohexylphosphino)-1-(2-methoxyphenyl)-1H-imidazol (24,2 mg; 66 μmol). Methanol (10 mL), Toluol (10 mL), 2-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 160 °C erwärmt und 40 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 89%. Die Selektivität vom C9-Amin beträgt 60% mit einem *n*/*i*-Verhältnis von 81:19. Die Selektivität vom linearen C9-Amin beträgt 49%. Außerdem werden 9% an *N*-Formylpiperidin gefunden.

*Beispiel 63 (Tabelle 7)*: Ein 100-mL Autoklav wird befüllt mit $Ru_3(CO)_{12}$ (25,6 mg; 40,0 μmol) und 2-(Dicyclohexylphosphino)-1-(2-methoxyphenyl)-1H-imidazol (48,4 mg; 132 μmol). Methanol (10 mL), Toluol (10 mL), 2-Okten (3,1 mL; 2,2 g; 20 mmol) und Piperidin (2,4 mL; 2,0 g; 24 mmol) werden hinzugegeben und es wird Synthesegas ($CO/H_2$ 1:5 6,0 MPa) aufgepresst. Die Reaktionsmischung wird auf 160 °C erwärmt und 30 Stunden bei dieser Temperatur gerührt. Danach wird der Autoklav abgekühlt und Gas abgelassen. Nach der Zugabe des internen Standards (Isooktan, 2,0 mL; 1,4 g; 12 mmol) wird die Reaktionsmischung gaschromatographisch analysiert. Der Umsatz von 1-Okten beträgt 94%. Die Selektivität vom C9-Amin beträgt 71% mit einem *n*/*i*-Verhältnis von 83:17. Die Selektivität vom linearen C9-Amin beträgt 59%. Außerdem werden 10% an *N*-Formylpiperidin gefunden.

Die angeführten Experimente zeigen deutlich, dass durch das erfindungsgemäße Verfahren ein hoch regioselektives und universell einsetzbares Verfahren zur Verfügung gestellt wird, welches gegenüber den bisher bekannten Verfahren auch noch kostengünstiger ist. Des Weiteren weist das direkte Verfahrensprodukt wenig *N*-Formylpiperidin auf.

## Patentansprüche

1. Verfahren zur Hydroaminomethylierung von Olefinen, **dadurch gekennzeichnet, dass** wenigstens ein Olefin mit mindestens einem primären oder sekundären Amin unter Zuführung von $H_2$ und CO in Gegenwart eines katalytischen

Systems aus einem Ruthenium-Komplex und mindestens einem Liganden umgesetzt wird, wobei der Ligand einen organischen PhosphorLiganden einer der allgemeinen Formeln (1) bis (8) darstellt:

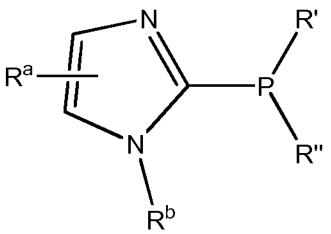
(1)

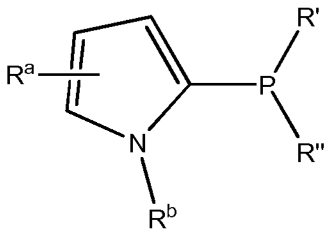
(2)

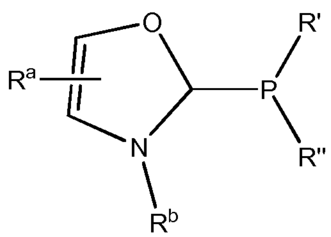
(3)

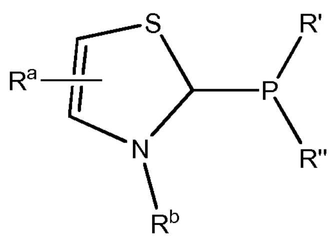
(4)

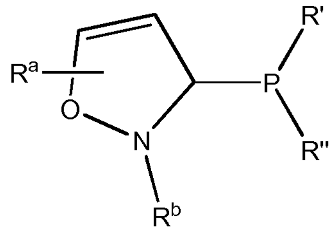
(5)

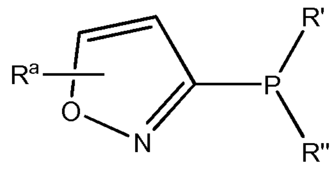
(6)

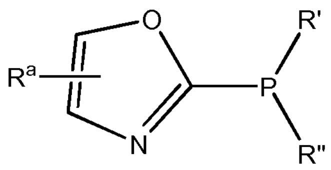
(7)

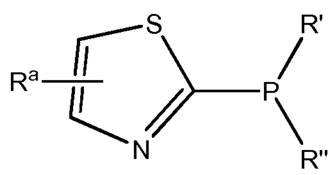
(8)

worin

R' und R" gleich oder verschieden sind und für einen Rest stehen, der ausgewählt ist aus der Gruppe bestehend aus:

$(C_1-C_{12})$-Alkyl, $(C_1-C_{12})$-Heteroalkyl, $(C_4-C_{14})$-Aryl, $(C_4-C_{14})$-Aryl-$(C_1-C_{12})$-Alkyl, $(C_4-C_{14})$-Aryl-O-$(C_1-C_{12})$-Alkyl, $(C_3-C_{14})$-Heteroaryl, $(C_3-C_{14})$-Heteroaryl-$(C_1-C_{12})$-Alkyl, $(C_3-C_{12})$-Cycloalkyl, $(C_3-C_{12})$-Cycloalkyl-$(C_1-C_{12})$-Alkyl, $(C_3-C_{12})$-Heterocycloalkyl, $(C_3-C_{12})$-Heterocycloalkyl-$(C_1-C_{12})$-Alkyl, O-$(C_1-C_{12})$-Alkyl, O-$(C_1-C_{12})$-Heteroalkyl, O-$(C_4-C_{14})$-Aryl, O-$(C_4-C_{14})$-Aryl-$(C_1-C_{14})$-Alkyl, O-$(C_3-C_{14})$-Heteroaryl, O-$(C_3-C_{14})$-Heteroaryl-$(C_1-C_{14})$-Alkyl, O-$(C_3-C_{12})$-Cycloalkyl, O-$(C_3-C_{12})$-Cycloalkyl-$(C_1-C_{12})$-Alkyl, O-$(C_3-C_{12})$-Heterocycloalkyl, O-$(C_3-C_{12})$-Heterocycloalkyl-$(C_1-C_{12})$-Alkyl,

wobei die genannten Alkyl-, Heteroalkyl-, Cycloalkyl-, Heterocycloalkyl-, Aryl- und Heteroarylgruppen optional ein oder mehrfach substituiert sind und
$R^a$ und $R^b$ ausgewählt sind aus: Wasserstoff, Hydroxy, $(C_1-C_{12})$-Alkyl, $(C_1-C_{12})$-Heteroalkyl, $(C_4-C_{14})$-Aryl, $(C_4-C_{14})$-Aryl-$(C_1-C_{12})$-Alkyl, $(C_4-C_{14})$-Aryl-O-$(C_1-C_{12})$-Alkyl, $(C_3-C_{14})$-Heteroaryl, $(C_3-C_{14})$-Heteroaryl-$(C_1-C_{12})$-Alkyl, $(C_3-C_{12})$-Cycloalkyl, $(C_3-C_{12})$-Cycloalkyl-$(C_1-C_{12})$-Alkyl, $(C_3-C_{12})$-Heterocycloalkyl, $(C_3-C_{12})$-Heterocycloalkyl-$(C_1-C_{12})$-Alkyl, O-$(C_1-C_{12})$-Alkyl, O-$(C_1-C_{12})$-Heteroalkyl, O-$(C_4-C_{14})$-Aryl, O-$(C_4-C_{14})$-Aryl-$(C_1-C_{14})$-Alkyl, O-$(C_3-C_{14})$-Heteroaryl, O-$(C_3-C_{14})$-Heteroaryl-$(C_1-C_{14})$-Alkyl, O-$(C_3-C_{12})$-Cyc-

loalkyl, O-($C_3$-$C_{12}$)-Cycloalkyl-($C_1$-$C_{12}$)-Alkyl, O-($C_3$-$C_{12}$)-Heterocycloalkyl, O-($C_3$-$C_{12}$)-Heterocycloalkyl-($C_1$-$C_{12}$)-Alkyl,

wobei die genannten Alkyl-, Heteroalkyl-, Cycloalkyl-, Heterocycloalkyl-, Aryl- und Heteroarylgruppen optional ein oder mehrfach substituiert sind und
und wobei $R^a$ auch zur Bildung eines größeren kondensierten Ringes befähigt ist.

2. Verfahren nach Anspruch 1,
wobei der Ligand die allgemeine Formel (1) oder (2) aufweist.

3. Verfahren nach Anspruch 1 oder 2,
wobei der Ligand die allgemeine Formel (1) aufweist.

4. Verfahren nach Anspruch 1 oder 2,
wobei der Ligand die allgemeine Formel (2) aufweist.

5. Verfahren nach einem der Ansprüche 1 bis 4,
wobei R' ausgewählt ist aus: Cyclohexyl, iso-Propyl, Phenyl.

6. Verfahren nach einem der Ansprüche 1 bis 5,
wobei R" ausgewählt ist aus: Cyclohexyl, iso-Propyl, Phenyl.

7. Verfahren nach einem der Ansprüche 1 bis 6,
**dadurch gekennzeichnet, dass** der Ligand ausgewählt ist aus der Gruppe bestehend aus:

2-(Dicyclohexylphosphino)-1-(2-methoxyphenyl)-1H-imidazol (L1),
2-(Dicyclohexylphosphino)-1-methyl-1H-imidazol (L2),
2-(Dicyclohexylphosphino)-1-phenyl-1H-imidazol (L3),
2-(Diisopropylphosphino)-1-(2-methoxyphenyl)-1H-imidazol (L4),
2-(Diphenylphosphino)-1-(2-methoxyphenyl)-1H-imidazol (L5),
2-(Dicyclohexylphosphino)-1-mesityl-1H-imidazol (L6),
2-(Dicyclohexylphosphino)-1-phenyl-1H-pyrrol (L7),
2-(Dicyclohexylphosphino)-1-(2-methoxyphenyl)-1H-pyrrol (L8),
2-(Dicyclohexylphosphino)-1-methyl-1H-benzo[d]imidazol (L9).

8. Verfahren nach einem der Ansprüche 1 bis 7,
**dadurch gekennzeichnet, dass** der Ruthenium-Katalysator in situ ausgehend von einem Vorkomplex gebildet wird, wobei als Rutheniumquelle Ruthenium-enthaltende Salze und Komplexe als Vorstufe verwendet werden, die Rutheniumcarbonylhydrid-Komplexe bilden.

9. Verfahren nach einem der Ansprüche 1 bis 8,
**dadurch gekennzeichnet, dass** es zur Umsetzung von Alkenen verwendet wird, die eine oder mehrere ethylenisch ungesättigte Doppelbindungen enthalten.

## Claims

1. Process for the hydroaminomethylation of olefins, **characterized in that** at least one olefin is reacted with at least one primary or secondary amine with the introduction of $H_2$ and CO, in the presence of a catalytic system of a ruthenium complex and at least one ligand, where the ligand is an organic phosphorus ligand of one of the general formulae (1) to (8):

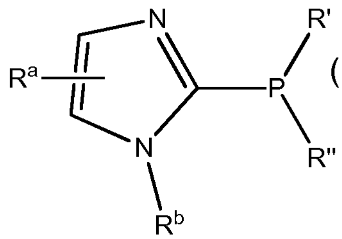
(1)

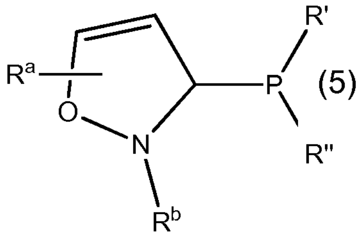
(5)

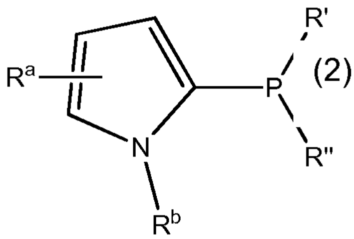
(2)

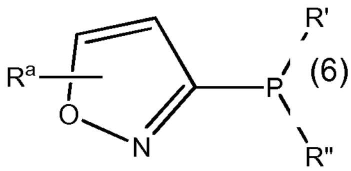
(6)

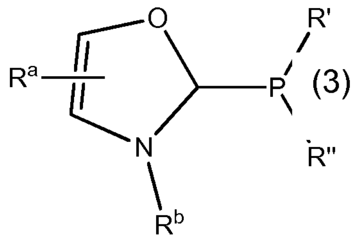
(3)

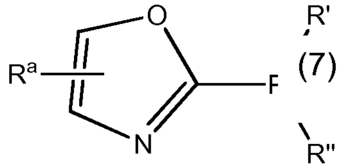
(7)

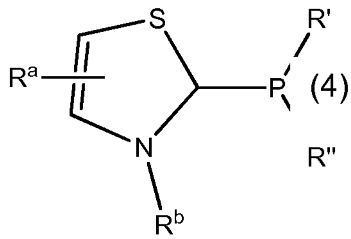
(4)

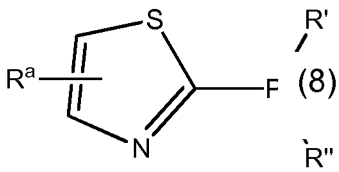
(8)

in which

R' and R" are identical or different and are a radical which is selected from the group consisting of:

$(C_1-C_{12})$-alkyl, $(C_1-C_{12})$ -heteroalkyl, $(C_4-C_{14})$-aryl, $(C_4-C_{14})$ -aryl- $(C_1-C_{12})$ -alkyl, $(C_4-C_{14})$-aryl-O-$(C_1-C_{12})$-alkyl, $(C_3-C_{14})$-heteroaryl, $(C_3-C_{14})$-heteroaryl-$(C_1-C_{12})$-alkyl, $(C_3-C_{12})$-cycloalkyl, $(C_3-C_{12})$-cycloalkyl-$(C_1-C_{12})$ -alkyl, $(C_3-C_{12})$-heterocycloalkyl, $(C_3-C_{12})$-heterocycloalkyl-$(C_1-C_{12})$-alkyl, O-$(C_1-C_{12})$-alkyl, O-$(C_1-C_{12})$-heteroalkyl, O-$(C_4-C_{14})$-aryl, O-$(C_4-C_{14})$-aryl-$(C_1-C_{14})$-alkyl, O-$(C_3-C_{14})$ -heteroaryl, O-$(C_3-C_{14})$ -heteroaryl- $(C_1-C_{14})$-alkyl, O-$(C_3-C_{12})$-cycloalkyl, O-$(C_3-C_{12})$-cycloalkyl-$(C_1-C_{12})$-alkyl, O-$(C_3-C_{12})$-heterocycloalkyl, O-$(C_3-C_{12})$ -heterocycloalkyl- $(C_1-C_{12})$ -alkyl,

where the specified alkyl, heteroalkyl, cycloalkyl, heterocycloalkyl, aryl and heteroaryl groups are optionally mono- or polysubstituted and
$R^a$ and $R^b$ are selected from: hydrogen, hydroxy, $(C_1-C_{12})$-alkyl, $(C_1-C_{12})$-heteroalkyl, $(C_4-C_{14})$-aryl, $(C_4-C_{14})$-aryl-$(C_1-C_{12})$-alkyl, $(C_4-C_{14})$-aryl-O-$(C_1-C_{12})$-alkyl, $(C_3-C_{14})$-heteroaryl, $(C_3-C_{14})$-heteroaryl-$(C_1-C_{12})$-alkyl, $(C_3-C_{12})$-cycloalkyl, $(C_3-C_{12})$-cycloalkyl-$(C_1-C_{12})$-alkyl, $(C_3-C_{12})$-heterocycloalkyl, $(C_3-C_{12})$-heterocycloalkyl-$(C_1-C_{12})$-alkyl, O-$(C_1-C_{12})$-alkyl, O-$(C_1-C_{12})$-heteroalkyl, O-$(C_4-C_{14})$-aryl, O-$(C_4-C_{14})$-aryl-$(C_1-C_{14})$-alkyl, O-$(C_3-C_{14})$ -heteroaryl, O-$(C_3-C_{14})$ -heteroaryl- $(C_1-C_{14})$-alkyl, O-$(C_3-C_{12})$-cycloalkyl, O-$(C_3-C_{12})$-cycloalkyl-$(C_1-C_{12})$-alkyl, O-$(C_3-C_{12})$-heterocycloalkyl, O-$(C_3-C_{12})$-heterocycloalkyl-$(C_1-C_{12})$-alkyl,

where the specified alkyl, heteroalkyl, cycloalkyl, heterocycloalkyl, aryl and heteroaryl groups are optionally mono- or polysubstituted and and where $R^a$ is also capable of forming a larger condensed ring.

**2.** Process according to Claim 1, where the ligand has the general formula (1) or (2).

**3.** Process according to Claim 1 or 2, where the ligand has the general formula (1).

**4.** Process according to Claim 1 or 2, where the ligand has the general formula (2).

**5.** Process according to one of Claims 1 to 4, where R' is selected from: cyclohexyl, isopropyl, phenyl.

**6.** Process according to one of Claims 1 to 5, where R" is selected from: cyclohexyl, isopropyl, phenyl.

**7.** Process according to one of Claims 1 to 6, **characterized in that** the ligand is selected from the group consisting of:

2-(dicyclohexylphosphino)-1-(2-methoxyphenyl)-1H-imidazole (L1),
2-(dicyclohexylphosphino)-1-methyl-1H-imidazole (L2),
2-(dicyclohexylphosphino)-1-phenyl-1H-imidazole (L3),
2-(diisopropylphosphino)-1-(2-methoxyphenyl)-1H-imidazole (L4),
2-(diphenylphosphino)-1-(2-methoxyphenyl)-1H-imidazole (L5),
2-(dicyclohexylphosphino)-1-mesityl-1H-imidazole (L6),
2-(dicyclohexylphosphino)-1-phenyl-1H-pyrrole (L7),
2-(dicyclohexylphosphino)-1-(2-methoxyphenyl)-1H-pyrrole (L8),
2-(dicyclohexylphosphino)-1-methyl-1H-benzo[d]imidazole (L9).

**8.** Process according to one of Claims 1 to 7, **characterized in that** the ruthenium catalyst is formed in situ starting from a precomplex, where, as ruthenium source, ruthenium-containing salts and complexes are used as precursor which form ruthenium carbonyl hydride complexes.

**9.** Process according to one of Claims 1 to 8, **characterized in that** it is used for the reaction of alkenes which contain one or more ethylenically unsaturated double bonds.

## Revendications

**1.** Procédé pour l'hydroaminométhylation d'oléfines, **caractérisé en ce qu'**on transforme au moins une oléfine avec au moins une amine primaire ou secondaire, avec addition de $H_2$ et de CO, en présence d'un système catalytique constitué par un complexe du ruthénium et au moins un ligand, le ligand étant un ligand phosphoré organique d'une des formules générales (1) à (8) :

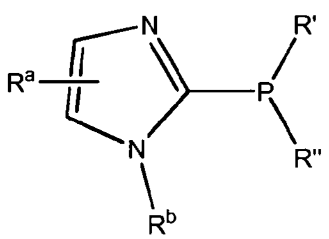
(1)

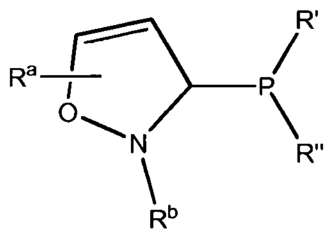
(5)

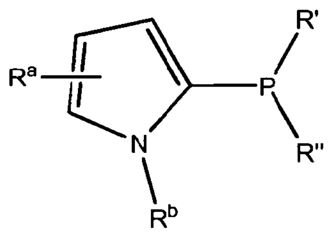
(2)

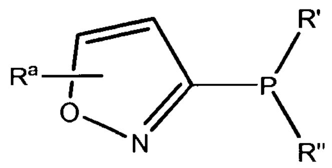
(6)

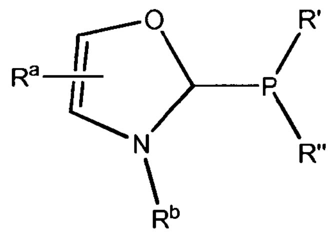
(3)

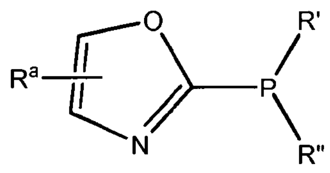
(7)

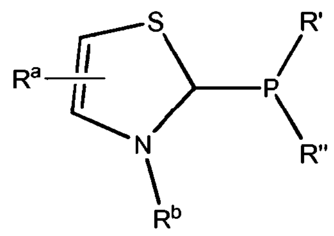
(4)

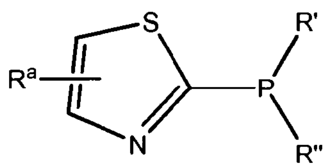
(8)

dans lesquelles

R' et R" sont identiques ou différents et représentent un radical qui est choisi dans le groupe constitué par :

$(C_1-C_{12})$-alkyle, $(C_1-C_{12})$-hétéroalkyle, $(C_4-C_{14})$-aryle, $(C_4-C_{14})$-aryl-$(C_1-C_{12})$-alkyle, $(C_4-C_{14})$-aryl-O-$(C_1-C_{12})$-alkyle, $(C_3-C_{14})$-hétéroaryle, $(C_3-C_{14})$-hétéroaryl- $(C_1-C_{12})$-alkyle, $(C_3-C_{12})$-cycloalkyle, $(C_3-C_{12})$-cycloalkyl-$(C_1-C_{12})$-alkyle, $(C_3-C_{12})$-hétérocycloalkyle, $(C_3-C_{12})$-hétérocycloalkyl-$(C_1-C_{12})$-alkyle, O-$(C_1-C_{12})$-alkyle, O-$(C_1-C_{12})$-hétéroalkyle, O-$(C_4-C_{14})$-aryle, O-$(C_4-C_{14})$-aryl- $(C_1-C_{14})$-alkyle, O-$(C_3-C_{14})$-hétéroaryle, O-$(C_3-C_{14})$-hétéroaryl-$(C_1-C_{14})$-alkyle, O-$(C_3-C_{12})$-cycloalkyle, O-$(C_3-C_{12})$-cycloalkyl-$(C_1-C_{12})$-alkyle, O-$(C_3-C_{12})$-hétérocycloalkyle, O-$(C_3-C_{12})$-hétérocycloalkyl-$(C_1-C_{12})$-alkyle, les groupes alkyle, hétéroalkyle, cycloalkyle, hétérocycloalkyle, aryle et hétéroaryle mentionnés étant éventuellement monosubstitués ou polysubstitués et

$R^a$ et $R^b$ sont choisis parmi : hydrogène, hydroxy, $(C_1-C_{12})$-alkyle, $(C_1-C_{12})$ -hétéroalkyle, $(C_4-C_{14})$ -aryle, $(C_4-C_{14})$ -aryl- $(C_1-C_{12})$ -alkyle, $(C_4-C_{14})$ -aryl-O-$(C_1-C_{12})$-alkyle, $(C_3-C_{14})$-hétéroaryle, $(C_3-C_{14})$-hétéroaryl-$(C_1-C_{12})$-alkyle, $(C_3-C_{12})$-cycloalkyle, $(C_3-C_{12})$-cycloalkyl-$(C_1-C_{12})$-alkyle, $(C_3-C_{12})$-hétérocycloalkyle, $(C_3-C_{12})$-hétérocycloalkyl-$(C_1-C_{12})$-alkyle, O-$(C_1-C_{12})$-alkyle, O-$(C_1-C_{12})$-hétéroalkyle, O-$(C_4-C_{14})$-aryle, O-$(C_4-C_{14})$-aryl- $(C_1-C_{14})$-alkyle, O-$(C_3-C_{14})$-hétéroaryle, O-$(C_3-C_{14})$-hétéroaryl-$(C_1-C_{14})$-alkyle, O-$(C_3-C_{12})$-cycloalkyle, O-$(C_3-C_{12})$-cycloalkyl-$(C_1-C_{12})$-alkyle, O-$(C_3-C_{12})$-hétérocycloalkyle, O-$(C_3-C_{12})$-hétérocycloalkyl-$(C_1-C_{12})$-alkyle,
les groupes alkyle, hétéroalkyle, cycloalkyle, hétérocycloalkyle, aryle et hétéroaryle mentionnés étant éventuellement monosubstitués ou polysubstitués et
et $R^a$ étant également apte à la formation d'un cycle condensé plus grand.

**2.** Procédé selon la revendication 1, le ligand présentant la formule générale (1) ou (2).

**3.** Procédé selon la revendication 1 ou 2, le ligand présentant la formule générale (1).

**4.** Procédé selon la revendication 1 ou 2, le ligand présentant la formule générale (2).

**5.** Procédé selon l'une quelconque des revendications 1 à 4, R' étant choisi parmi : cyclohexyle, isopropyle, phényle.

**6.** Procédé selon l'une quelconque des revendications 1 à 5, R" étant choisi parmi : cyclohexyle, isopropyle, phényle.

**7.** Procédé selon l'une quelconque des revendications 1 à 6, **caractérisé en ce que** le ligand est choisi dans le groupe constitué par :

le 2-(dicyclohexylphosphino)-1-(2-méthoxyphényl)-1H-imidazole (L1),
le 2-(dicyclohexylphosphino)-1-méthyl-1H-imidazole (L2),
le 2-(dicyclohexylphosphino)-1-phényl-1H-imidazole (L3),
le 2-(diisopropylphosphino)-1-(2-méthoxyphényl)-1H-imidazole (L4),
le 2-(diphénylphosphino)-1-(2-méthoxyphényl)-1H-imidazole (L5),
le 2-(dicyclohexylphosphino)-1-mésityl-1H-imidazole (L6),
le 2-(dicyclohexylphosphino)-1-phényl-1H-pyrrole (L7),
le 2-(dicyclohexylphosphino)-1-(2-méthoxyphényl)-1H-pyrrole (L8),
le 2-(dicyclohexylphosphino)-1-méthyl-1H-benzo [d] imidazole (L9).

**8.** Procédé selon l'une quelconque des revendications 1 à 7, **caractérisé en ce que** le catalyseur du ruthénium est formé in situ partant d'un précomplexe, des sels et des complexes contenant du ruthénium étant utilisés comme source de ruthénium en tant que précurseurs, qui forment des complexes d'hydrure de ruthénium-carbonyle.

**9.** Procédé selon l'une quelconque des revendications 1 à 8, **caractérisé en ce qu'**il est utilisé pour la transformation d'alcènes, qui contiennent une ou plusieurs doubles liaisons éthyléniquement insaturées.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- DE 10321421 A1
- WO 2007078859 A2


### In der Beschreibung aufgeführte Nicht-Patentliteratur


- **REPPE, W. ; VETTER, H.** *Liebigs Ann. Chem.,* 1953, vol. 582, 133-163
- **EINE VON L. F. TIETZE.** *Chem. Rev.,* 1996, vol. 96, 115-136
- **G. H. POSNER.** *Chem. Rev.,* 1986, vol. 86, 831-844
- **EILBRACHT, P. ; KRANEMANN, C. L. ; BÄRFACKER, L.** *Eur. J. Org. Chem,* 1999, 1907-1914
- **RISCHE, T. ; BÄRFACKER, L. ; EILBRACHT, P.** *Eur. J. Org. Chem,* 1999, 653-660
- **KOÇ, F. ; WYSZOGRODZKA, M. ; EILBRACHT, P. ; HAAG, R.** *J. Org. Chem.,* 2005, vol. 70, 2021-2025
- **BEIGI, M. ; RICKEN, S. ; MUELLER, K. S. ; KOC, F. ; EILBRACHT, P.** *Eur. J. Org. Chem,* 2011, 1482-1492
- **SUBHANI, M. A. ; MUELLER, K.-S. ; EILBRACHT, P.** *Adv. Synth. Catal.,* 2009, vol. 351, 2113-2123
- **SEAYAD, A. ; AHMED, M. ; KLEIN, H. ; JACKSTELL, R. ; GROSS, T. ; BELLER, M.** *Science,* 2002, vol. 297, 1676-1678
- **AHMED, M. ; SEAYAD, A. M. ; JACKSTELL, R. ; BELLER, M.** *J. Am. Chem. Soc,* 2003, vol. 125, 10311-10318
- **AHMED, M. ; BRONGER, R. P. J. ; JACKSTELL, R. ; KAMER, P. C. J. ; VAN LEEUWEN, P. W. N. M. ; BELLER, M.** *Chem. Eur. J.,* 2006, vol. 12, 8979-88
- **AHMED, M. ; BUCH, C. ; ROUTABOUL, L. ; JACKSTELL, R. ; KLEIN, H. ; SPANNENBERG, A. ; BELLER, M.** *Chem. Eur. J.,* 2007, vol. 13, 1594-1601
- **LIU, G. ; HUANG, K. ; CAI, C. ; CAO, B. ; CHANG, M. ; WU, W. ; ZHANG, X.** *Chem. Eur. J.,* 2011, vol. 17, 14559-14563
- **LIU, G. ; HUANG, K. ; CAO, B. ; CHANG, M. ; LI, S. ; YU, S. ; ZHOU, L. ; WU, W. ; ZHANG, X.** *Org. Lett.,* 2012, vol. 14, 102-105
- **FREDIANI, P. ; BIANCHI, M. ; SALVINI, A. ; CARLUCCIO, L. C. ; ROSI, L.** *J. Organomet. Chem.,* 1997, vol. 547, 35-40
- **HAYASHI, T. ; HUI GU, Z. ; SAKAKURA, T. ; TANAKA, M.** *J. Organomet. Chem.,* 1988, vol. 352, 373-378
- **KHAN, M. M. T. ; HALLIGUDI, S. B. ; ABDI, S. H. R.** *J. Mol. Catal. A: Chem.,* 1988, vol. 48, 313-317
- **MITSUDO, T.-A. ; SUZUKI, N. ; KONDO, T. ; WATANABE, Y.** *J. Mol. Catal. A: Chem.,* 1996, vol. 109, 219-225
- Methoden wie sie in Chemistry of Organophosphorous Compounds. John Whiley, 1990, vol. 1
- **ZHANG, H. ; CAI, Q. ; MA, D.** *J. Org. Chem.,* 2005, vol. 70 (13), 5164-5173
- **SCHULZ, T. ; TORBORG, C. ; SCHÄFFNER, B. ; HUANG, J. ; ZAPF, A. ; KADYROV, R. ; BÖRNER, A. ; BELLER, M.** *Angew. Chem. Int. Ed.,* 2009, vol. 48, 918-921